# EUROPEAN PATENT APPLICATION

(11) **EP 4 378 956 A1**
(43) Date of publication of application: **05.06.2024**
(21) Application number: 22903484.8
(22) Date of filing: 07.12.2022
(51) Int. Cl.: C07K 16/28, C12N 15/13, A61K 39/395, A61K 47/68, A61P 35/00, A61P 37/06, A61P 31/00, C12N 5/07, C12N 15/11, C12N 15/63, G01N 33/68, G01N 33/577, G01N 33/574

(54) **B7H6 ANTIBODY AND USE THEREOF**

(30) Priority: 07.12.2021 CN 202111485380
(71) Applicant: Hefei TG Immunopharma Co., Ltd., Hefei, Anhui 230001 (CN)
(72) Inventor: TIAN, Zhigang, Hefei, Anhui 230001 (CN); CAO, Guoshuai, Hefei, Anhui 230001 (CN); XIAO, Weihua, Hefei, Anhui 230001 (CN); SUN, Rui, Hefei, Anhui 230001 (CN); SUN, Haoyu, Hefei, Anhui 230001 (CN)
(74) Representative: Latscha Schöllhorn Partner AG
(86) International application number: PCT/CN2022/137095
(87) International publication number: WO 2023/104062

(57) **Abstract**

The present disclosure discloses a B7H6 antibody and use thereof. The antibody includes heavy chain CDR1, heavy chain CDR2 and heavy chain CDR3; and light chain CDR1, light chain CDR2 and light chain CDR3. The antibody of the present disclosure is capable of binding to B7H6, and promoting the binding of B7H6 to the NK cell activating receptor NKp30, with an anti-cancer function.

## Description

### PRIORITY INFORMATION

This application claims priority and the benefit of the patent application No. 202111485380.9 submitted to the China National Intellectual Property Administration on December 7, 2021, which is incorporated herein by reference in its entirety.

### FIELD

The present disclosure relates to the field of antibodies and, in particular, to an antibody capable of binding to B7H6 or antigen binding fragment thereof and the use thereof.

### BACKGROUND

NK cell is a very important innate lymphocyte in the body, which plays an important role in anti-virus and anti-tumor. NK cells express a variety of activating receptors on their surface, including NKp30, NKG2D, etc., and recognize and bind to ligands expressed on the surface of the tumor cell. These ligands are encoded by their own genes and expressed on the cell surface under conditions such as malignant transformation (tumor) of cells.

NKp30 is an important NK cell activating receptor, which transmits an activation signal to promote the NK cell to kill the tumor after binding to its ligand. B7H6 is the only known NKp30 ligand expressed as a membrane protein. B7-H6 is highly expressed in a variety of tumor tissues, such as melanoma and liver cancer, but not expressed in normal tissues, and has tumor specificity.

TGF-β and other cytokines in the tumor microenvironment down-regulate the expression of NKp30 on the surface of the NK cell, which results in the failure of NK cells to utilize the NKp30-B7H6 interaction to accurately recognize and kill tumors, thus leading to tumor immune escape. Therefore, it is necessary to provide a therapeutic antibody drug targeting B7H6.

### SUMMARY

The object of the present disclosure is to overcome the deficiencies of the prior art and to provide a B7H6 antibody capable of binding to human and monkey B7H6 and promoting the binding of B7H6 to NKp30, and uses thereof.

The antibody provided in the present disclosure can promote the anti-cancer function of NK cells by promoting the binding of B7H6 to NKp30.

In one aspect, the present disclosure provides an antibody or antigen binding fragment thereof capable of binding B7H6, wherein the antibody or antigen binding fragment thereof includes a heavy chain CDR1, a heavy chain CDR2, a heavy chain CDR3, a light chain CDR1, a light chain CDR2, and a light chain CDR3, wherein
the heavy chain CDR1 includes at least one amino acid sequence selected from the group consisting of an amino acid sequence as set forth in SEQ ID NO. 1, and SEQ ID NO. 7, and an amino acid sequence in conservative modification form thereof;
the heavy chain CDR2 includes at least one amino acid sequence selected from the group consisting of an amino acid sequence as set forth in SEQ ID NO. 2, and SEQ ID NO. 8, and an amino acid sequence in conservative modification form thereof;
the heavy chain CDR3 includes at least one amino acid sequence selected from the group consisting of an amino acid sequence as set forth in SEQ ID NO. 3, and SEQ ID NO. 9, and an amino acid sequence in conservative modification form thereof;
the light chain CDR1 includes at least one amino acid sequence selected from the group consisting of an amino acid sequence as set forth in SEQ ID NO. 4, and SEQ ID NO. 10, and an amino acid sequence in conservative modification form thereof;
the light chain CDR2 includes at least one amino acid sequence selected from the group consisting of an amino acid sequence as set forth in SEQ ID NO. 5, and SEQ ID NO. 11, and an amino acid sequence in conservative modification form thereof; and
the light chain CDR3 includes at least one amino acid sequence selected from the group consisting of an amino acid sequence as set forth in SEQ ID NO. 6, and SEQ ID NO. 12, and an amino acid sequence in conservative modification form thereof.

In certain embodiments, the antibody or antigen binding fragment thereof includes:
1) the heavy chain CDR1 having an amino acid sequence as set forth in SEQ ID NO. 1, the heavy chain CDR2 having an amino acid sequence as set forth in SEQ ID NO. 2, the heavy chain CDR3 having an amino acid sequence as set forth in SEQ ID NO. 3, the light chain CDR1 having an amino acid sequence as set forth in SEQ ID NO. 4, the light chain CDR2 having an amino acid sequence as set forth in SEQ ID NO. 5, and the light chain CDR3 having an amino acid sequence as set forth in SEQ ID NO. 6: or
2) the heavy chain CDR1 having an amino acid sequence as set forth in SEQ ID NO. 7, the heavy chain CDR2 having an amino acid sequence as set forth in SEQ ID NO. 8, the heavy chain CDR3 having an amino acid sequence as set forth in SEQ ID NO. 9, the light chain CDR1 having an amino acid sequence as set forth in SEQ ID NO. 10, the light chain CDR2 having an amino acid sequence as set forth in SEQ ID NO. 11, and the light chain CDR3 having an amino acid sequence as set forth in SEQ ID NO. 12.

In certain embodiments, the antibody or antigen binding fragment thereof includes a heavy chain variable region and a light chain variable region, (i) the heavy chain variable region includes an amino acid sequence that is at least 80% homologous to at least one of an amino acid sequence as set forth in SEQ ID NO. 13, SEQ ID NO. 15, SEQ ID NO. 17, SEQ ID NO. 19 and an amino acid sequence in conservative modification form thereof; and/or, (ii) the light chain variable region includes an amino acid sequence that is at least 80% homologous to at least one of an amino acid sequence as set forth in SEQ ID NO. 14, SEQ ID NO. 16, SEQ ID NO. 18, SEQ ID NO. 20 and an amino acid sequence in conservative modification form thereof.

In certain embodiments, the heavy chain variable region includes an amino acid sequence that is at least 90%, at least 95%, at least 96%, at least 97%, at least 98%, at least 99%, or 100% homologous to the heavy chain variable region selected from (i); wherein the light chain variable region includes an amino acid sequence that is at least 90%, at least 95%, at least 96%, at least 97%, at least 98%, at least 99%, or 100% homologous to the light chain variable region selected from (ii).

In certain embodiments, the antibody or antigen binding fragment thereof includes:
1) an amino acid sequence of the heavy chain variable region as set forth in SEQ ID NO. 13, and an amino acid sequence of the light chain variable region as set forth in SEQ ID NO. 14;
2) an amino acid sequence of the heavy chain variable region as set forth in SEQ ID NO. 15, and an amino acid sequence of the light chain variable region as set forth in SEQ ID NO. 16;
3) an amino acid sequence of the heavy chain variable region as set forth in SEQ ID NO. 17, and an amino acid sequence of the light chain variable region as set forth in SEQ ID NO. 18; or
4) an amino acid sequence of the heavy chain variable region as set forth in SEQ ID NO. 19, and an amino acid sequence of the light chain variable region as set forth in SEQ ID NO. 20.

In certain embodiments, the antibody or antigen binding fragment thereof includes
1) an amino acid sequence of the heavy chain as set forth in SEQ ID NO. 26, and an amino acid sequence of the light chain as set forth in SEQ ID NO. 27;
2) an amino acid sequence of the heavy chain as set forth in SEQ ID NO. 28, and an amino acid sequence of the light chain as set forth in SEQ ID NO. 29;
3) an amino acid sequence of the heavy chain as set forth in SEQ ID NO. 30, and an amino acid sequence of the light chain as set forth in SEQ ID NO. 31; or
4) an amino acid sequence of the heavy chain as set forth in SEQ ID NO. 32, and an amino acid sequence of the light chain as set forth in SEQ ID NO. 33.

In certain embodiments, the isolated antibody is IgG1, IgG2, or IgG4.

In certain embodiments, the antibody is a monoclonal antibody, murine antibody, chimeric antibody, humanized antibody, human engineered antibody, human antibody, Fv, single chain antibody (scFv), Fab, Fab', Fab'-SH, or F(ab')2.

In certain embodiments, the antibody or antigen binding fragment thereof is capable of binding to the amino acid sequence as set forth in SEQ ID NO. 21.

In another aspect, the present disclosure provides an immunoconjugate including a therapeutic agent and the antibody or the antigen binding fragment thereof described above conjugated to the therapeutic agent.

In yet another aspect, the present disclosure provides a composition including the antibody or the antigen binding fragment thereof described above, and/or the immunoconjugate described above, and a pharmaceutically acceptable carrier.

In yet still another aspect, the present disclosure provides a kit for detecting B7H6 in a sample, the kit includes the antibody or antigen binding fragment thereof described above.

In yet still another aspect, the present disclosure provides the use of the antibody or antigen binding fragment thereof described above in preparation of a reagent for detection of B7H6 in a sample.

In yet still another aspect, the present disclosure provides the use of the antibody or antigen binding fragment thereof described above in preparation of a medicament for prevention and/or treatment of a B7H6-mediated disease, wherein the B7H6-mediated disease is a cancer or an infectious disease.

Optionally, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

In yet another aspect, the present disclosure provides a nucleic acid including a nucleic acid encoding the antibody or the antigen binding fragment thereof described above.

In a further aspect, the present disclosure provides a recombinant vector or transformant including the nucleic acid described above.

In one aspect, the present disclosure provides a recombinant cell carrying a nucleic acid, expressing the vector or transformant described above or expressing the antibody or the antigen binding fragment thereof described above.

In another aspect, the present disclosure provides a medicament including the antibody or antigen binding fragment thereof, immunoconjugate, composition, medicament, nucleic acid, recombinant vector or transformant, or recombinant cell described above.

In a further aspect, the present disclosure provides the use of the antibody or antigen binding fragment thereof, immunoconjugate, composition, medicament, nucleic acid, recombinant vector or transformant or recombinant cell described above in the treatment and/or prevention of a B7H6-mediated disease. As described above, the antibody or antigen binding fragment thereof is capable of effectively binding to B7H6, thereby promoting the interaction of B7H6 with NKp30 and enhancing the anti-cancer function of NK cells, so that the antibody or antigen binding fragment thereof and the substances capable of expressing the antibody or antigen binding fragment thereof under appropriate conditions can effectively prevent and/or treat a B7H6-mediated disease.

In another aspect, the present disclosure provides a method for treating and/or preventing a B7H6-mediated disease. According to an embodiment of the present disclosure, the method includes administering to the subject at least one of 1) the antibody or antigen binding fragment thereof described above; 2) the immunoconjugate described above; 3) the composition described above; 4) the medicament described above; 5) the nucleic acid described above; 6) the recombinant vector or transformant described above; and 7) the recombinant cell described above. As described above, the antibody or antigen binding fragment thereof can effectively bind to B7H6, thereby promoting the interaction of B7H6 with NKp30 and enhancing the anti-cancer function of NK cells, so that the antibody or antigen binding fragment thereof, and the substances capable of expressing the antibody or antigen binding fragment thereof under appropriate conditions, or the substances including the antibody or antigen binding fragment thereof can effectively prevent and/or treat a B7H6-mediated disease, and the method according to the embodiment of the present disclosure can effectively prevent and/or treat a B7H6-mediated disease.

In yet another aspect, the present disclosure provides a method for diagnosing whether a subject has a B7H6-mediated disease. According to an embodiment of the present disclosure, the method includes detecting B7H6 in a sample to be tested using at least one of the following: 1) the antibody or antigen binding fragment thereof described above; 2) the nucleic acid described above; 3) the recombinant vector or transformant described above; and 4) the recombinant cell described above; and determining the content of B7H6 in the sample to be tested based on the detection result of the B7H6. As previously described, the antibody or antigen binding fragment of an embodiment of the present disclosure is capable of effectively binding to B7H6, and therefore, the antibody or antigen binding fragment can be used to detect B7H6, and B7H6 mediate a variety of diseases, and therefore, whether a subject has a B7H6-mediated disease can be determined based on the level of the B7H6 contained in a biological sample from the subject. In addition, the above-mentioned substances can also be used for monitoring the content of B7H6 in the sample to be tested of a subject, namely, the above-mentioned substances can also be used for disease staging of a subject suffering from a B7H6-mediated disease, and can also be used for prognosis of a B7H6-mediated disease.

In yet another aspect, the present disclosure provides use of at least one of following for diagnosing whether a subject has a B7H6-mediated disease: 1) the antibody or antigen binding fragment thereof described above; 2) the nucleic acid described above; 3) the recombinant vector or transformant described above; and 4) the recombinant cell described above. As previously described, the antibody or antigen binding fragment of an embodiment of the present disclosure is capable of effectively binding to B7H6, and therefore, the antibody or antigen binding fragment can be used to detect B7H6, and B7H6 mediate a variety of diseases, and therefore, whether a subject has a B7H6-mediated disease can be determined based on the level of the B7H6 contained in a biological sample from the subject.

In accordance with the above technical solutions, the present disclosure provides an anti-B7H6 antibody or antigen binding fragment thereof capable of binding to human and monkey B7H6. The antibody or antigen binding fragment thereof of the present disclosure has at least one of the following properties: capable of binding to human and monkey B7H6; capable of promoting the interaction between B7H6 and NKp30; capable of promoting the anti-cancer function of NK cells.

Additional features and advantages of the present disclosure will be set forth in the detailed description which follows.

Additional aspects and advantages of the present disclosure will be set forth in part in the description which follows and, in part, will be apparent from the following description, or may be learned by practice of the present disclosure.

### BRIEF DESCRIPTION OF DRAWINGS

The foregoing and/or additional aspects and advantages of the present disclosure will become apparent and readily appreciated from the following description of the embodiments, taken in conjunction with the accompanying drawings of which:

The accompanying drawings are intended to provide a further understanding of the present disclosure and form a part of the specification. The drawings, together with the specific embodiments below are used to explain the present invention but do not constitute a limitation of the present disclosure. In the drawings:
FIG. 1 is a graph of ELISA showing the binding of a murine B7H6 antibody to human B7H6 according to a specific embodiment of the present disclosure;
FIG. 2 is a graph showing the binding of a murine B7H6 antibody to HL60-B7H6 cells according to a specific embodiment of the present disclosure;
FIG. 3 is a graph of ELISA showing the binding of a humanized B7H6 antibody to human B7H6 according to a specific embodiment of the present disclosure;
FIG. 4 is a graph showing the binding of a humanized B7H6 antibody to HL60-B7H6 cells according to a specific embodiment of the present disclosure;
FIG. 5 is a graph showing the binding of a humanized B7H6 antibody to CHO-K1-cyno B7H6 cells according to a specific embodiment of the present disclosure;
FIG. 6 is a graph of ELISA showing the binding of humanized B7H6 antibody to monkey B7H6 according to a specific embodiment of the present disclosure;
FIG. 7 is a graph showing the effect of a B7H6 antibody in promoting the binding of receptor NKp30 according to a specific embodiment of the present disclosure;
FIG. 8 is a graph showing the complement dependent cytotoxic effect mediated by a B7H6 antibody according to a specific embodiment of the present disclosure; and
FIG. 9 is a graph showing the effect of a B7H6 antibody in promoting the antagonizing human acute myeloid leukemia U-937 tumor in nude mice according to a specific embodiment of the present disclosure.

### DESCRIPTION OF EMBODIMENTS

Hereinafter, the embodiments of the present disclosure will be described in detail, embodiments of which are illustrated in the accompanying drawings. The embodiments described below with reference to the figures are exemplary and are intended to explain the present disclosure and are not to be construed as limiting the present disclosure.

It should be noted that the terms "first" and "second" are used for descriptive purposes only and are not to be construed as indicating or implying relative importance or implicitly indicating the number of technical features indicated. Thus, a feature defined with "first" or "second" may explicitly or implicitly includes one or more of the features. Further, in the description of the present disclosure, unless otherwise specified, the meaning of "a plurality" is two or more.

Hereinafter, specific embodiments of the present disclosure will be described in detail. It should be understood that the specific embodiments described herein are only used to illustrate and explain the present disclosure and are not to limit the present disclosure.

The endpoints of the ranges and any values disclosed herein are not limited to the precise range or value, and such ranges or values should be understood to include values approximating such ranges or values. For numerical ranges, the endpoints of each range, the endpoints of each range and the individual point values, and the individual point values, can be combined with each other to produce one or more new numerical ranges, and such numerical ranges are to be considered to be specifically disclosed herein.

For an easier understanding of the present disclosure, certain technical and scientific terms are specifically defined below. Unless clearly defined elsewhere in this document, all other technical and scientific terms used herein have the meanings commonly understood by general technical personnel in the field to which the present disclosure belongs. Abbreviations for amino acid residues are standard 3-letter and/or 1-letter codes used in the art to refer to one of the 20 commonly used L-amino acids.

In the present disclosure, when not stated to the contrary, the term antigen binding fragment, i.e. "antibody fragment" used generally refers to an antigen binding antibody fragment and may include a portion of an intact antibody, typically an antigen binding or variable region. Examples of antibody fragments include Fab, Fab', F(ab')2, Fv or scFv, diabody, linear antibody, single-chain antibody molecules, etc.

The term "complementarity determining region" or "CDR" or "CDR sequence" refers to the amino acid sequence responsible for antigen binding in an antibody, for example, generally includes amino acid residues near 23-34 (L1), 50-56 (L2) and 89-97 (L3) in the light chain variable region, and 31-35B (H1), 50-65 (H2) and 95-102 (H3) in the heavy chain variable region (Kabat et al. Sequences of Proteins of Immunological Interest, 5th Ed. Public Health Service, National Institutes of Health, Bethesda, MD.(1991)); and/or amino acid residues from the "hypervariable loops" (e.g. near 26-32 (LI), 50-52 (L2), and 91-96 (L3) in the light chain variable region, and 26-32 (H1), 53-55 (H2), and 96-101 (H3) in the heavy chain variable region (Chothia and Lesk J.Mol.Biol. 196: 901-917(1987)).

The term "an amino acid sequence in conservative modification form" refers to an amino acid modification that does not significantly affect or alter the binding characteristics of an antibody including the amino acid sequence, including amino acid substitutions, additions and deletions. Modifications can be introduced into the antibodies of the present disclosure by standard techniques such as site-directed mutagenesis and PCR-mediated mutagenesis. Conservative amino acid substitution refers to the substitution of amino acid residues by amino acid residues with similar side chains. Families of amino acid residues having similar side chains have been identified in the art. These families include amino acids with basic side chains, e.g. lysine, arginine, histidine, amino acids with acidic side chains, e.g. aspartic acid, glutamic acid, amino acids with uncharged polar side chains, e.g. glycine, asparagine, glutamine, serine, threonine, tyrosine, cysteine, tryptophan, amino acids with nonpolar side chains, e.g. alanine, valine, leucine, isoleucine, proline, phenylalanine, methionine, amino acids with β-branched side chains, e.g. threonine, valine, isoleucine, and amino acids with aromatic side chains, e.g. tyrosine, phenylalanine, tryptophan, histidine. Thus, one or more of the amino acid residues in the CDR region of an antibody of the present disclosure can be replaced with other amino acid residues from the same side chain family, and the altered antibody can be tested for retained function using the functional assays described herein. Preferably, the number of conservative modifications does not exceed one or two.

For polypeptides, the term "(substantial) homology" means that at least about 80%, usually at least about 90% to 95%, and more preferably at least about 98% to 99.5% of the amino acids of two polypeptides or designated sequences thereof are identical when optimally aligned and compared (in which nucleotides are inserted or deleted appropriately).

The % identity between two sequences varies with the number of identical positions shared by the sequences when the sequences are optimally aligned (i.e.% homology = number of identical positions/total number of positions × 100), where optimal alignment is determined taking into account the number of gaps that need to be introduced and the length of each gap to achieve optimal alignment of the two sequences. Sequence comparison and percent identity determination between two sequences can be accomplished using mathematical algorithms, as described in the non-limiting examples below.

One skilled in the art may substitute, add and/or delete one or more (e.g. 1, 2, 3, 4, 5, 6, 7, 8, 9, or 10 or more) amino acids to a sequence of the present disclosure to obtain a variant of the sequence of the antibody or functional fragment thereof without substantially affecting the activity of the antibody (retaining at least 95% activity). They are considered to be included within the scope of the present disclosure. For example, amino acids having similar properties may be substituted in the variable region. The sequence of the variant of the present disclosure may have at least 80%, 85%, 90%, 95%, 96%, 97%, 98%, or 99% identity (or homology) to the reference sequence. Sequence identity described herein can be measured using sequence analysis software, e.g. a computer program BLAST using default parameters, in particular BLASTP or TBLASTN. Amino acid sequences referred to herein are shown from the N-terminus to the C-terminus.

As previously described, the antibody of the present disclosure may be full length (e.g. IgG1 or IgG4 antibodies) or may include only an antigen binding portion (e.g. Fab, F(ab')2 or scFv fragment), or may be modified to affect function. The present disclosure includes anti-B7H6 antibodies having modified glycosylation patterns. In some applications, it may be useful to make modifications to remove undesired glycosylation sites, or to eliminate the presence of fucose moieties on the oligosaccharide chains, e.g. to enhance antibody-dependent ADCC function. In other applications, galactosylation modifications can be made to alter complement dependent cytotoxicity (CDC).

The term "functional fragment" as used herein refers in particular to a partial fragment of an antibody such as Fv, scFv (sc means single chain), Fab, F(ab')2, Fab', scFv-Fc fragment or diabody, or any fragment which should be capable of increasing the half-life by chemical modification, e.g. addition of polyalkylene glycols, such as polyethylene glycol ("PEGylation, PEG") (referred to as pegylated fragments of Fv-PEG, scFv-PEG, Fab-PEG, F(ab')2-PEG or Fab'-PEG) ("PEG" means polyethylene glycol) or by incorporation into liposomes, said fragments having B7H6 binding activity. Preferably, the functional fragment will consist of or contain partial sequences of the heavy chain variable region or light chain variable region of its source antibody, sufficient to retain the same binding specificity and sufficient affinity as its source antibody. For B7H6, it is preferred to be at least 1/100 of its source antibody affinity, and in a more preferred manner, at least 1/10. Such function fragments will include a minimum of 5 amino acids, preferably 10, 15, 25, 50, and 100 contiguous amino acids of the antibody sequence from which they are derived.

As described above, the present disclosure provides an antibody capable of binding to B7H6 or antigen binding fragment thereof, wherein the antibody includes heavy chain CDR1, heavy chain CDR2, and heavy chain CDR3; light chain CDR1, light chain CDR2, and light chain CDR3.

According to the present disclosure, the antibody is capable of binding to B7H6, particularly the amino acid sequence shown in SEQ ID NO. 21.

In a preferred embodiment of the present disclosure, in order to further improve the biological acceptability of the antibody, the antibody may also be humanized, i.e. the antibody is a chimeric or humanized antibody. The term "chimeric antibody" refers to a recombinant antibody obtained by replacing the amino acid sequence of the constant region of a monoclonal antibody from one species (e.g. mouse) with the constant region of an antibody from another species (e.g. human) using recombinant DNA technology. The term "humanized antibody" refers to a recombinant antibody obtained by completely replacing the constant and the non-CDR (Fv framework region (FR)) amino acid sequences of variable regions of a monoclonal antibody from one species (e.g. mouse) with the constant and the non-CDR amino acid sequences of variable regions of an antibody from another species (e.g. human) using recombinant DNA technology. That is, when the constant region of an antibody is humanized, it is referred to as a chimeric antibody, and the constant and the non-CDR amino acid sequences of variable regions are all humanized, it is referred to as a humanized antibody. Methods of humanization may be carried out with reference to conventional antibody engineering techniques and will not be described in detail herein.

The sequence of the heavy chain variable region of the humanized antibody provided by the present disclosure is as set forth in SEQ ID NO. 17, and the sequence of the light chain variable region is as set forth in SEQ ID NO. 18; alternatively, the sequence of the heavy chain variable region is as set forth in SEQ ID NO. 19 and the sequence of the light chain variable region is as set forth in SEQ ID NO. 20.

The immunoconjugate provided by the present disclosure includes a therapeutic agent and the antibody or antigen binding fragment thereof described above conjugated to the therapeutic agent. The manner in which the antibody or antigen binding fragment thereof is conjugated to the therapeutic agent can be conventional.

The composition provided by the present disclosure includes the antibody or antigen binding fragment thereof described above, and/or the immunoconjugate described above, and a pharmaceutically acceptable carrier. In certain embodiments, the composition includes combinations that are separated in time and/or space so long as they can work together to achieve the objects of the present disclosure. For example, the components contained in the composition can be administered to subjects as a whole or separately. When the components contained in the composition are administered separately to a subject, the individual components may be administered to the subject simultaneously or sequentially.

The term "pharmaceutically acceptable" indicates that the composition can be administered to a subject without causing an adverse physiological reaction that would preclude the administration of the composition. For example, "pharmaceutically acceptable carrier" refers to a carrier that is useful in preparing a generally safe, non-toxic, and desirable pharmaceutical composition. Preferably, examples of such carriers or diluents include, but are not limited to water, saline, Ringer's solution, dextrose, mannitol, glucose, lactose, starch, magnesium stearate, cellulose, magnesium carbonate, 0.3% glycerol, hyaluronic acid, ethanol, polyalkylene glycols such as polypropylene glycol, triglycerides, 5% human serum albumin; liposomes and non-aqueous vehicles such as fixed oils may also be used.

The composition of the present disclosure may also be administered in combination with each other, or combination with one or more other therapeutic compounds, for example, in combination with a chemotherapeutic agent. Thus, the composition may also contain the chemotherapeutic agent. The antibody or antibody binding fragment thereof, or immunoconjugate of the present disclosure may also be combined with a second therapeutic agent, exemplary agents of which include, but are not limited to, other agents that inhibit B7H6 activity (including other antibodies or antigen binding fragments thereof, peptide inhibitors, small molecule antagonists, etc.) and/or agents that interfere with B7H6 upstream or downstream signal transduction.

Typically, the antibody or antigen binding fragment thereof is administered in an effective amount, i.e. an amount sufficient to achieve the desired therapeutic and/or prophylactic effect, e.g. an amount that results in the prevention or amelioration of symptoms associated with the disease being treated, e.g. a disease associated with B7H6. The effective amount of the composition administered to a subject will depend on the type and severity of the disease, as well as on the characteristics of the individual, such as general health, age, sex, weight, and drug tolerance; it will also depend on the severity and type of disease, and one skilled in the art will be able to determine appropriate dosages depending on such factors, etc.

The kit for detecting B7H6 in a sample provided by the present disclosure includes the antibody or antigen binding fragment thereof described above. The sample may be a tissue of a patient suffering from a B7H6-mediated disease, in particular a transplant rejection, autoimmune disease, infectious disease, or cancer patient, more preferably a patient suffering from at least one of the lung cancer, liver cancer, ovarian cancer, cervical cancer, skin cancer, bladder cancer, colon cancer, breast cancer, glioma, kidney cancer, gastric cancer, esophageal cancer, oral squamous cell cancer and head and neck cancer. The kit may also include reagents conventionally used to detect B7H6, such as coating solutions and the like.

The disclosure also provides the use of the antibody or antigen binding fragment thereof described above in preparation of a reagent for the detection of B7H6 in a sample. As previously mentioned, the sample may be the tissue from a patient suffering from a B7H6-mediated disease, which is not described in detail herein. The antibody or antigen binding fragment thereof of the present disclosure has a good affinity with B7H6 and is capable of effectively detecting B7H6 in a sample.

The disclosure also provides the use of the antibody or antigen binding fragment thereof, immunoconjugate, composition, nucleic acid, recombinant vector or transformant or recombinant cell for the preparation of a medicament for the prevention and/or treatment of a B7H6-mediated disease. Preferably, the B7H6-mediated disease is a transplant rejection, an autoimmune disease, an infectious disease, or a cancer. More preferably, the cancer is a cancer expressing B7H6. Further preferably, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer. Further preferably, the infectious disease includes but is not limited to, HIV virus infection and/or hepatitis B virus infection.

The present disclosure relates to a medicament including the antibody or antigen binding fragment thereof, immunoconjugate, composition, nucleic acid, recombinant vector, or transformant or recombinant cell described above.

According to some embodiments of the present disclosure, the medicament may include at least one of the following additional technical features:
According to some embodiments of the present disclosure, the medicament includes a pharmaceutically acceptable carrier and an effective amount of the antibody or antigen binding fragment thereof, immunoconjugate, composition, nucleic acid, recombinant vector, or transformant or recombinant cell.

The present disclosure also relates to a method for preventing and/or treating a B7H6-mediated disease (described above), the method including: administering to a patient an effective amount of at least one of the antibody or antigen binding fragment thereof, immunoconjugate, composition, recombinant cell, nucleic acid, and medicament of the present disclosure. The mode of administration may be oral, nasal, intradermal, subcutaneous, intramuscular or intravenous, or intraperitoneal. As previously described, the antibody or antigen binding fragment thereof is capable of effectively binding to B7H6, thereby promoting the interaction of B7H6 with NKp30 and enhancing NK cell anti-cancer function. Thus, the antibody or antigen binding fragment thereof, the substances capable of expressing the antibody or antigen binding fragment thereof under appropriate conditions, or the substances including the antibody or antigen binding fragment thereof can effectively prevent and/or treat a B7H6-mediated disease, and the method according to the embodiment of the present disclosure can effectively prevent and/or treat a B7H6-mediated disease.

According to some specific embodiments of the present disclosure, the method may further include at least one of the following additional technical features:
According to a specific embodiment of the present disclosure, the B7H6-mediated disease is cancer or an infectious disease.

According to a specific embodiment of the present disclosure, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

As used herein, "patient" or "subject" refers generally to mammals, such as primates and/or rodents, particularly humans or mice.

One skilled in the art can clone a DNA molecule encoding an antibody or antigen binding fragment thereof of the present disclosure into a vector, particularly an expression vector, and transform a host cell to obtain the antibody or antigen binding fragment thereof by inducing expression. Thus, the present disclosure also provides an (isolated) nucleic acid encoding the antibody or antigen binding fragment thereof described above, as well as a recombinant vector and a transformant containing the same. The nucleic acid is preferably an expression cassette obtained by genetic engineering means.

A recombinant vector may refer to either a cloning vector or an expression vector, and can be obtained by operably linking the nucleic acid to a commercially available vector (e.g. a plasmid or viral vector). Common plasmids include pSeTag2, PEE14, pMH3, and the like.

The expression vectors of the present disclosure may contain DNA sequences encoding the heavy chain variable region, the light chain variable region, and/or the constant region of the antibody. However, it is also possible to construct two expression vectors respectively, one containing heavy chain variable and constant regions and the other containing light chain variable and constant regions, and transfect the mammalian cells together. In a preferred embodiment, the expression vector further includes a promoter and a DNA sequence encoding a secretion signal peptide, and at least one drug resistance gene for screening.

The host cell of the present disclosure may be a prokaryotic host cell, a eukaryotic host cell, or a bacteriophage. The prokaryotic host cell may be *E. coli*, *Bacillus subtilis*, *Streptomyces* or *Proteus mirabilis*, etc. The eukaryotic cells may be fungi such as *Pichia pastoris*, *Saccharomyces cerevisiae*, *Schizosaccharomyces pombe*, *Trichoderma*, insect cells such as *Mythimna separata*, plant cells such as tobacco, mammalian cells such as BHK cells, CHO cells, COS cells, myeloma cells, and the like. In some embodiments, the host cells of the present disclosure are preferably mammalian cells, more preferably BHK cells, CHO cells, NSO cells, or COS cells.

The present disclosure relates to a recombinant cell carrying the nucleic acid, recombinant vector or transformant, or antibody or antigen binding fragment described above. The recombinant cell is obtained by transfecting or transforming the expression vector. According to an embodiment of the present disclosure, the recombinant cell highly expresses the above-mentioned antibody under suitable conditions, the antibody or antigen binding fragment thereof can effectively bind to B7H6, and further, the antibody or antigen binding fragment has a good effect in preventing and/or treating a B7H6-mediated disease.

It is to be noted that the recombinant cell of the present disclosure is not particularly limited and may be a prokaryotic cell, a eukaryotic cell, or a bacteriophage. The prokaryotic cell may be *E. coli, Bacillus subtilis*, *Streptomyces* or *Proteus mirabilis*, etc. The eukaryotic cells may be fungi such as *Pichia pastoris*, *Saccharomyces cerevisiae*, *Schizosaccharomyces pombe*, *Trichoderma*, insect cells such as *Mythimna separata*, plant cells such as tobacco, mammalian cells such as BHK cells, CHO cells, COS cells, myeloma cells, and the like. In some embodiments, the recombinant cells of the present disclosure are preferably mammalian cells, including BHK cells, CHO cells, NSO cells, or COS cells, and do not include animal germ cells, fertilized eggs, or embryonic stem cells.

It should be noted that "suitable conditions" as used in the description of the present application refers to conditions suitable for the expression of the antibody or the antigen binding fragment thereof described herein. Those skilled in the art will readily appreciate that conditions suitable for expression of the antibody or antigen binding fragment include, but are not limited to, suitable transformation or transfection manners, suitable transformation or transfection conditions, a healthy host cell state, a suitable host cell density, a suitable cell culture environment, and a suitable cell culture time. The "suitable conditions" are not particularly limited, and one skilled in the art can optimize the optimal conditions for expression of the antibody or antigen binding fragment thereof according to the particular circumstances of the laboratory.

The disclosure also relates to the use of the antibody or antigen binding fragment thereof, immunoconjugate, composition, medicament, nucleic acid, recombinant vector, or transformant or recombinant cell described above in treatment and/or prevention of a B7H6-mediated disease. As described above, the antibody or antigen binding fragment thereof can effectively bind to B7H6, thereby promoting the interaction of B7H6 with NKp30 and enhancing the anti-cancer function of NK cells, so that the antibody or antigen binding fragment thereof and the substances capable of expressing the antibody or antigen binding fragment thereof under appropriate conditions all can effectively prevent and/or treat a B7H6-mediated disease.

According to particular an embodiment of the present disclosure, the above-mentioned use in the treatment and/or prevention of a B7H6-mediated disease may further include at least one of the following additional technical features:
According to a specific particular embodiment of the present disclosure, the B7H6-mediated disease is a cancer or an infectious disease.

According to a specific embodiment of the present disclosure, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

In yet another aspect of the present disclosure, the present disclosure provides a method for diagnosing whether a subject has a B7H6-mediated disease. According to a specific embodiment of the present disclosure, the method includes detecting B7H6 in a sample to be tested using at least one of the following: 1) the antibody or antigen binding fragment thereof described above; 2) the nucleic acid described above; 3) the recombinant vector or transformant described above; and 4) the recombinant cell described above; and determining the content of B7H6 in the sample to be tested based on a result of detecting the B7H6. As previously described, the antibody or antigen binding fragment of an embodiment of the present disclosure is capable of effectively binding to B7H6, and therefore, the antibody or antigen binding fragment can be used to detect B7H6, and B7H6 mediate a variety of diseases, and therefore, whether a subject has a B7H6-mediated disease can be determined based on the level of the B7H6 contained in a biological sample from the subject. In addition, the above-mentioned substances can also be used for monitoring the content of B7H6 in the sample to be tested of a subject, namely, the above-mentioned substances can also be used for disease staging of a subject suffering from a B7H6-mediated disease, and can also be used for prognosis of a B7H6-mediated disease.

According to a specific embodiment of the present disclosure, the above method may further include at least one of the following additional technical features:
According to a specific embodiment of the present disclosure, a content of B7H6 in the sample to be tested that is not lower than the minimum standard for the disease is an indication that the sample to be tested is derived from a patient suffering from a B7H6-mediated disease. The value of the minimum standard can be determined by comparing, analyzing and verifying the difference in the content of B7H6 in the sample to be tested from a large number of individuals with the B7H6-mediated disease and a large number of healthy individuals.

According to a specific embodiment of the present disclosure, the B7H6-mediated disease is a cancer or an infectious disease.

According to a specific embodiment of the present disclosure, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

According to a specific embodiment of the present disclosure, the sample to be tested includes at least one of the following: tissue, cells, blood, serum, sweat, feces, and urine.

In another aspect of the present disclosure, the present disclosure provides use of at least one of in diagnosing whether a subject has a B7H6-mediated disease: 1) the antibody or antigen binding fragment thereof described above; 2) the nucleic acid described above; 3) the recombinant vector or transformant described above; and 4) the recombinant cell described above. As previously described, the antibody or antigen binding fragment of an embodiment of the present disclosure is capable of effectively binding to B7H6, and therefore, the antibody or antigen binding fragment can be used to detect B7H6, and B7H6 mediate a variety of diseases, and therefore, whether a subject has a B7H6-mediated disease can be determined based on the level of the B7H6 contained in a biological sample from the subject.

According to a specific embodiment of the present disclosure, the above uses may further include at least one of the following additional technical features:
According to a specific embodiment of the present disclosure, the B7H6-mediated disease is a cancer or an infectious disease.

According to a specific embodiment of the present disclosure, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

A description of the sequences involved in the present disclosure is given in Table 1.

**Table 1 List of amino acid sequences**

| SEQ ID NO. | Sequence | Description |
|---|---|---|
| 1 | GYTFTSYY | 12F5 HCDR1 |
| 2 | INPSNGGT | 12F5 HCDR2 |
| 3 | TRTLYYGNDWYFDV | 12F5 HCDR3 |
| 4 | SSVNY | 12F5 LCDR1 |
| 5 | DTS | 12F5 LCDR2 |
| 6 | QQWSSNPVT | 12F5 LCDR3 |
| 7 | GYTFTDYN | 12G4 HCDR1 |
| 8 | INPNNGGT | 12G4 HCDR2 |
| 9 | ARSEVFYGNYADY | 12G4 HCDR3 |
| 10 | QSVDYDGDSY | 12G4 LCDR1 |
| 11 | AAS | 12G4 LCDR2 |
| 12 | QQSKEDPRT | 12G4 LCDR3 |
| 13 | QVQLQQSGAELVKPGASVKLSCKASGYTFTSYYMYWVKQRPGQGLEWI GEINPSNGGTNFNEKFKSKATLTVDKSSSTAYMQLSSLTSEDSAVYYCTRTL YYGNDWYFDVWGAGTTVTVSS | Murine 12F5 heavy chain variable region |
| 14 | QIVLTQSPAIMSASPGEKVTMTCSASSSVNYMHWYQQKSGTSPKRWIYDT SKLASGVPARFSGSGSGTSYSLTISSMEAEDAATYYCQQWSSNPVTFGAGT KLELK | Murine 12F5 light chain variable region |
| 15 | EVLLQQSGPEVVKPGASVKITCKASGYTFTDYNMDWVKQSHGKSLEWIG DINPNNGGTLYNQKFRGRVTLIVDKSSSTAYMELRSLTSDDTAVYYCARSE VFYGNYADYWGQGTTLTVSS | Murine 12G4 heavy chain variable region |
| 16 | DIVLTQSPVSLAVPLGQRATISCKASQSVDYDGDSYMNWYQQKPGQPPKL LIYAASTLHSGIPVRFSGSGSGTDFTLNIHPVEEEDAASYYCQQSKEDPRTF GGGTKLEIK | Murine 12G4 light chain variable region |
| 17 | EVQLVQSGAEVKKPGASVKVSCKASGYTFTSYYMYWVRQAPGQGLEWI GEINPSNGGTNFNEKFKSRVTLTVDKSISTAYMELSRLRSDDTAVYYCTRTL YYGNDWYFDVWGQGTTVTVSS | Humanized 12F5 heavy chain variable region |
| 18 | EIVLTQSPDFQSVTPKEKVTITCSASSSVNYMHWYQQKPDQSPKRLIYDTS KLASGVPSRFSGSGSGTDYTLTINSLEAEDAATYYCQQWSSNPVTFGQGTK LEIK | Humanized 12F5 light chain variable region |
| 19 | EVQLVQSGAEVKKPGASVKVSCKASGYTFTDYNMDWVRQAPGKGLEWI GDINPNNGGTLYNQKFRGRVTLTVDKSISTAYMELSRLRSDDTAVYYCARS EVFYGNYADYWGQGTTVTVSS | Humanized 12G4 heavy chain variable region |
| 20 | DIVLTQTPLSLSVTPGQPASISCKASQSVDYDGDSYMNWYLQKPGQPPQLL IYAASTLHSGVPDRFSGSGSGTDFTLKISRVEAEDVGVYYCQQSKEDPRTF GQGTKLEIK | Humanized 12G4 light chain variable region |
| 21 | MTWRAAASTCAALLILLWALTTEGDLKVEMMAGGTQITPLNDNVTIFCNI FYSQPLNITSMGITWFWKSLTFDKEVKVFEFFGDHQEAFRPGAIVSPWRLK SGDASLRLPGIQLEEAGEYRCEVVVTPLKAQGTVQLEVVASPASRLLLDQ VGMKENEDKYMCESSGFYPEAINITWEKQTQKFPHPIEISEDVITGPTIKN MDGTFNVTSCLKLNSSQEDPGTVYQCVVRHASLHTPLRSNFTLTAARHSL SETEKTDNFSIHWWPISFIGVGLVLLIVLIPWKKICNKSSSAYTPLKCILKHW NSFDTQTLKKEHLIFFCTRAWPSYQLQDGEAWPPEGSVNINTIQQLDVFCR QEGKWSEVPYVQAFFALRDNPDLCQCCRIDPALLTVTSGKSIDDNSTKSEK QTPREHSDAVPDAPILPVSPIWEPPPATTSTTPVLSSQPPTLLLPLQ | B7H6 protein |
| 22 | DLKVEMMAGGTQITPLNDNVTIFCNIFYSQPLNITSMGITWFWKSLTFDKE VKVFEFFGDHQEAFRPGAIVSPWRLKSGDASLRLPGIQLEEAGEYRCEVV VTPLKAQGTVQLEVVASPASRLLLDQVGMKENEDKYMCESSGFYPEAINI TWEKQTQKFPHPIEISEDVITGPTIKNMDGTFNVTSCLKLNSSQEDPGTVY QCVVRHASLHTPLRSNFTLTAARHSLSETEKTDNFSLEPKSCDKTHTCPPC PAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKAL PAPIEKTISKAKGQPREPQVYTLPPSREEMTKNQVSLTCLVKGFYPSDIAVE WESNGQPENNYKTTPPVLDSDGSFFLYSKLTVDKSRWQQGNVFSCSVMH EALHNHYTQKSLSLSPGK | Recombinant B7H6 extracellular region Fc fusion protein |
| 23 | DLKVEMMARGIQATRLNDSVTISCKVIYSQPLNITSMGITWFRKSLTLDKE VKVFEFFGDHQKAFRPGANVSLWRLKSGDASLKLPGVQLEEAGEYRCEV VVTPLKAQGTVQLKVVASPTSRLFQDQAVVKENEGKYMCESSRFYPKDIN ITWEKWTQKSPHHVVISENVITGPTIKNMDGTFNITSYLKLNSSQEDPGTV YRCVIRHESLHTPVSIDFILTAPQQSLSEPEKTDPKSCDKTHTCPPCPAPELL GGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEK TISKAKGQPREPQVYTLPPSRDELTKNQVSLTCLVKGFYPSDIAVEWESNG QPENNYKTTPPVLDSDGSFFLYSKLTVDKSRWQQGNVFSCSVMHEALHN HYTQKSLSLSPGK | Recombinant Cynomolgus monkey B7H6 extracellular region Fc fusion protein |
| 24 | MAWRAASSACAAPLLLWCALMTAGDLKVEMMARGIQATRLNDSVTISCK VIYSQPLNITSMGITWFRKSLTLDKEVKVFEFFGDHQKAFRPGANVSLWRL KSGDASLKLPGVQLEEAGEYRCEVVVTPLKAQGTVQLKVVASPTSRLFQD QAVVKENEGKYMCESSRFYPKDINITWEKWTQKSPHHVVISENVITGPTIK NMDGTFNITSYLKLNSSQEDPGTVYRCVIRHESLHTPVSIDFILTAPQQSLSE PEKTDIFSIYGWLFSFIAAGLILLIVLIHWKKVPLREEEGRN | Cynomolgus monkey B7H6 protein |
| 25 | WVSQPPEIRTLEGSSAFLPCSFNASQGRLAIGSVTWFRDEVVPGKEVRNGT PEFRGRLAPLASSRFLHDHQAELHIRDVRGHDASIYVCRVEVLGLGVGTG NGTRLVVEKEHPQLGENLYFQSLEPKSCDKTHTCPPCPAPELLGGPSVFLFP PKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQP REPQVYTLPPSREEMTKNQVSLTCLVKGFYPSDIAVEWESNGQPENNYKTT PPVLDSDGSFFLYSKLTVDKSRWQQGNVFSCSVMHEALHNHYTQKSLSLS PGK | Recombinant NKp30 extracellular region Fc fusion protein |
| 26 | QVQLQQSGAELVKPGASVKLSCKASGYTFTSYYMYWVKQRPGQGLEWI GEINPSNGGTNFNEKFKSKATLTVDKSSSTAYMQLSSLTSEDSAVYYCTRTL YYGNDWYFDVWGAGTTVTVSS AKTTAPSVYPLAPVCGDTTGSSVTLGCLVKGYFPEPVTLTWNSGSLSSGVH TFPAVLQSDLYTLSSSVTVTSSTWPSQSITCNVAHPASSTKVDKKIEPRGPTI KPCPPCKCPAPNLLGGPSVFIFPPKIKDVLMISLSPIVTCVVVDVSEDDPDV QISWFVNNVEVHTAQTQTHREDYNSTLRVVSALPIQHQDWMSGKEFKCK VNNKDLPAPIERTISKPKGSVRAPQVYVLPPPEEEMTKKQVTLTCMVTDF MPEDIYVEWTNNGKTELNYKNTEPVLDSDGSYFMYSKLRVEKKNWVER NSYSCSVVHEGLHNHHTTKSFSRTPGK | Murine 12F5 heavy chain |
| 27 | QIVLTQSPAIMSASPGEKVTMTCSASSSVNYMHWYQQKSGTSPKRWIYDT SKLASGVPARFSGSGSGTSYSLTISSMEAEDAATYYCQQWSSNPVTFGAGT KLELKRADAAPTVSIFPPSSEQLTSGGASVVCFLNNFYPKDINVKWKIDGS | Murine 12F5 light chain |
| | ERQNGVLNSWTDQDSKDSTYSMSSTLTLTKDEYERHNSYTCEATHKTSTS PIVKSFNRNEC | |
| 28 | EVLLQQSGPEVVKPGASVKITCKASGYTFTDYNMDWVKQSHGKSLEWIG DINPNNGGTLYNQKFRGRVTLIVDKSSSTAYMELRSLTSDDTAVYYCARSE VFYGNYADYWGQGTTLTVSSAKTTAPSVYPLAPVCGDTTGSSVTLGCLVK GYFPEPVTLTWNSGSLSSGVHTFPAVLQSDLYTLSSSVTVTSSTWPSQSITC NVAHPASSTKVDKKIEPRGPTIKPCPPCKCPAPNLLGGPSVFIFPPKIKDVLM ISLSPIVTCVVVDVSEDDPDVQISWFVNNVEVHTAQTQTHREDYNSTLRVV SALPIQHQDWMSGKEFKCKVNNKDLPAPIERTISKPKGSVRAPQVYVLPPP EEEMTKKQVTLTCMVTDFMPEDIYVEWTNNGKTELNYKNTEPVLDSDGS YFMYSKLRVEKKNWVERNSYSCSVVHEGLHNHHTTKSFSRTPGK | Murine 12G4 heavy chain |
| 29 | DIVLTQSPVSLAVPLGQRATISCKASQSVDYDGDSYMNWYQQKPGQPPKL LIYAASTLHSGIPVRFSGSGSGTDFTLNIHPVEEEDAASYYCQQSKEDPRTF GGGTKLEIKRADAAPTVSIFPPSSEQLTSGGASVVCFLNNFYPKDINVKWKI DGSERQNGVLNSWTDQDSKDSTYSMSSTLTLTKDEYERHNSYTCEATHKT STSPIVKSFNRNEC | Murine 12G4 light chain |
| 30 | EVQLVQSGAEVKKPGASVKVSCKASGYTFTSYYMYWVRQAPGQGLEWI GEINPSNGGTNFNEKFKSRVTLTVDKSISTAYMELSRLRSDDTAVYYCTRTL YYGNDWYFDVWGQGTTVTVSS ASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSC DKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYK CKVSNKALPAPIEKTISKAKGQPREPQVYTLPPSREEMTKNQVSLTCLVKG FYPSDIAVEWESNGQPENNYKTTPPVLDSDGSFFLYSKLTVDKSRWQQGN VFSCSVMHEALHNHYTQKSLSLSPGK | Humanized 12F5 heavy chain |
| 31 | EIVLTQSPDFQSVTPKEKVTITCSASSSVNYMHWYQQKPDQSPKRLIYDTS KLASGVPSRFSGSGSGTDYTLTINSLEAEDAATYYCQQWSSNPVTFGQGTK LEIKRTVAAPSVFIFPPSDEQLKSGTASVVCLLNNFYPREAKVQWKVDNAL QSGNSQESVTEQDSKDSTYSLSSTLTLSKADYEKHKVYACEVTHQGLSSPV TKSFNRGEC | Humanized 12F5 light chain |
| 32 | EVQLVQSGAEVKKPGASVKVSCKASGYTFTDYNMDWVRQAPGKGLEWI GDINPNNGGTLYNQKFRGRVTLTVDKSISTAYMELSRLRSDDTAVYYCARS EVFYGNYADYWGQGTTVTVSS ASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSC DKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYK CKVSNKALPAPIEKTISKAKGQPREPQVYTLPPSREEMTKNQVSLTCLVKG FYPSDIAVEWESNGQPENNYKTTPPVLDSDGSFFLYSKLTVDKSRWQQGN VFSCSVMHEALHNHYTQKSLSLSPGK | Humanized 12G4 heavy chain |
| 33 | DIVLTQTPLSLSVTPGQPASISCKASQSVDYDGDSYMNWYLQKPGQPPQLL IYAASTLHSGVPDRFSGSGSGTDFTLKISRVEAEDVGVYYCQQSKEDPRTF GQGTKLEIKRTVAAPSVFIFPPSDEQLKSGTASVVCLLNNFYPREAKVQWK VDNALQSGNSQESVTEQDSKDSTYSLSSTLTLSKADYEKHKVYACEVTHQ GLSSPVTKSFNRGEC | Humanized 12G4 light chain |

The nucleic acid encoding the heavy and/or light chains of the antibodies of the present disclosure within the scope of the present disclosure, according to the amino acid sequences of the heavy and/or light chains, the corresponding nucleic acid sequences can be readily obtained by the person skilled in the art, as shown in Table 2.

**Table 2 List of nucleotide sequences**

| SEQ ID NO. | Sequence | Description |
|---|---|---|
| 34 | GGGTACACATTTACCAGCTACTAT | 12F5 HCDR1 |
| 35 | ATTAATCCATCTAACGGGGGAACC | 12F5 HCDR2 |
| 36 | ACTAGGACCCTGTATTACGGTAACGACTGGTATTTCGATGTG | 12F5 HCDR3 |
| 37 | TCTTCTGTTAACTAT | 12F5 LCDR1 |
| 38 | GACACCTCC | 12F5 LCDR2 |
| 39 | CAGCAATGGAGCAGTAATCCAGTCACA | 12F5 LCDR3 |
| 40 | GGGTACACATTTACAGACTACAAT | 12G4 HCDR1 |
| 41 | ATCAATCCAAACAATGGCGGTACC | 12G4 HCDR2 |
| 42 | GCTCGCTCTGAAGTCTTTTATGGGAATTATGCTGACTAC | 12G4 HCDR3 |
| 43 | CAGAGCGTGGACTATGATGGCGACAGCTAT | 12G4 LCDR1 |
| 44 | GCCGCCTCC | 12G4 LCDR2 |
| 45 | CAGCAGAGCAAAGAGGATCCTAGAACC | 12G4 LCDR3 |
| 46 | CAGGTGCAGTTGCAACAATCTGGTGCAGAGCTGGTGAAACCTGGTGCA TCAGTGAAGCTTTCATGTAAAGCTTCCGGGTACACATTTACCAGCTACTA TATGTACTGGGTTAAGCAGCGTCCTGGACAGGGTCTGGAGTGGATCGGC GAGATTAATCCATCTAACGGGGGAACCAACTTTAACGAAAAGTTCAAGT CTAAGGCTACACTGACAGTCGATAAATCCTCATCCACCGCCTACATGCA GCTCTCTTCTCTGACTTCAGAGGACAGCGCCGTGTACTACTGCACTAGG ACCCTGTATTACGGTAACGACTGGTATTTCGATGTGTGGGGTGCCGGTAC AACAGTGACCGTGTCCTCA | Murine 12F5 heavy chain variable region |
| 47 | CAAATCGTGCTGACCCAGTCTCCAGCAATAATGTCCGCATCTCCTGGTG AGAAAGTGACCATGACTTGTTCAGCTTCCTCTTCTGTTAACTATATGCAC TGGTACCAACAGAAGTCTGGCACCTCCCCAAAGCGGTGGATTTATGACA CCTCCAAGCTGGCTAGCGGGGTACCCGCAAGGTTCTCCGGTTCAGGCA GCGGCACTTCTTACTCCCTGACTATTAGCTCTATGGAGGCAGAGGATGCC GCTACCTACTATTGCCAGCAATGGAGCAGTAATCCAGTCACATTTGGGG CCGGAACCAAGCTGGAGCTGAAG | Murine 12F5 light chain variable region |
| 48 | GAAGTTCTGCTTCAGCAGTCTGGCCCAGAAGTTGTTAAGCCAGGAGCC TCCGTGAAAATTACTTGCAAGGCCTCCGGGTACACATTTACAGACTACA ATATGGACTGGGTGAAGCAGTCCCATGGAAAGAGTCTGGAATGGATCGG CGATATCAATCCAAACAATGGCGGTACCCTCTATAACCAGAAATTTCGCG GACGTGTGACCTTGATCGTCGACAAAAGTAGTTCTACTGCCTACATGGA GCTGCGCAGCCTGACTAGTGACGACACCGCCGTCTACTATTGCGCTCGC TCTGAAGTCTTTTATGGGAATTATGCTGACTACTGGGGCCAGGGTACCAC TTTGACCGTCTCCAGT | Murine 12G4 heavy chain variable region |
| 49 | GACATTGTGCTGACTCAGAGTCCTGTAAGTCTGGCAGTTCCCCTCGGCC AAAGAGCTACCATTAGTTGTAAGGCTTCCCAGAGCGTGGACTATGATGG CGACAGCTATATGAATTGGTATCAACAGAAGCCAGGACAGCCACCCAAG CTGCTGATCTACGCCGCCTCCACCCTGCATTCAGGTATCCCTGTGCGATT TTCTGGTAGTGGCTCCGGCACAGATTTCACCCTGAATATCCATCCAGTTG AGGAGGAGGATGCTGCCTCTTACTACTGTCAGCAGAGCAAAGAGGATC CTAGAACCTTCGGCGGTGGCACTAAGCTGGAAATTAAG | Murine 12G4 light chain variable region |
| 50 | GAAGTTCAACTGGTTCAGAGCGGAGCTGAGGTAAAGAAGCCAGGTGCC AGCGTGAAAGTTAGTTGCAAGGCATCCGGATACACTTTCACTAGCTATT ATATGTATTGGGTGCGACAGGCCCCAGGGCAAGGTTTGGAGTGGATCGG AGAGATCAATCCTTCAAATGGCGGCACAAATTTCAACGAAAAGTTCAA ATCTCGCGTGACTCTGACAGTGGACAAGTCCATCTCCACAGCATATATG GAGCTGAGCCGGCTTAGATCCGATGACACTGCTGTCTACTATTGTACAA GAACACTGTACTATGGCAACGACTGGTACTTCGATGTCTGGGGACAGGG GACCACTGTGACCGTCTCCAGT | Humanized 12F5 heavy chain variable region |
| 51 | GAAATCGTGCTGACCCAGAGCCCAGATTTCCAGAGTGTCACACCAAAG GAGAAGGTAACAATTACATGCAGTGCCAGCAGTAGCGTGAACTATATGC ATTGGTATCAGCAGAAACCCGATCAGTCCCCTAAGCGACTGATCTACGA TACCTCCAAGCTGGCCAGCGGTGTTCCAAGTAGGTTCTCTGGCAGCGGG TCTGGGACAGACTACACTCTCACTATCAACTCTCTGGAAGCTGAGGATG CTGCTACTTATTATTGCCAGCAGTGGAGCTCAAATCCCGTTACCTTTGGC | Humanized 12F5 light chain variable region |
| | CAGGGTACCAAGCTCGAAATTAAA | |
| 52 | GAAGTGCAGCTTGTACAGAGTGGAGCAGAGGTCAAGAAACCAGGAGC TTCCGTGAAGGTGAGCTGCAAGGCCTCCGGCTATACCTTTACTGATTAC AACATGGACTGGGTCCGTCAGGCTCCTGGAAAAGGACTGGAGTGGATT GGTGACATTAATCCTAATAACGGCGGCACACTTTACAATCAGAAGTTTC GTGGTAGAGTTACACTGACTGTGGATAAATCAATCAGCACCGCCTATATG GAGCTGTCCCGACTGCGGTCCGACGATACTGCAGTGTATTACTGCGCCA GGTCAGAGGTGTTTTACGGTAACTACGCAGACTATTGGGGCCAGGGGAC AACAGTGACAGTCAGCAGC | Humanized 12G4 heavy chain variable region |
| 53 | GACATAGTATTGACCCAGACACCATTGTCTTTGAGCGTTACCCCAGGCC AGCCAGCTTCAATATCTTGCAAGGCCTCTCAGTCCGTCGACTATGATGGC GATTCCTACATGAATTGGTACCTCCAAAAGCCAGGCCAACCACCACAGC TGTTGATTTATGCCGCTTCTACACTCCACAGTGGCGTGCCCGATCGATTT TCAGGATCCGGCTCAGGGACCGATTTCACCCTTAAGATTTCTCGAGTGG AAGCCGAGGATGTGGGCGTGTATTACTGCCAGCAGAGCAAAGAGGATC CTAGAACTTTTGGCCAGGGAACCAAGCTGGAGATTAAA | Humanized 12G4 light chain variable region |
| 54 | ATGACGTGGAGGGCTGCCGCCTCCACGTGCGCGGCGCTCCTGATTCTGC TGTGGGCGCTGACGACCGAAGGTGATCTGAAAGTAGAGATGATGGCAG GGGGGACTCAGATCACACCCCTGAATGACAATGTCACCATATTCTGCAA TATCTTTTATTCCCAACCCCTCAACATCACGTCTATGGGTATCACCTGGTT TTGGAAGAGTCTGACGTTTGACAAAGAAGTCAAAGTCTTTGAATTTTTT GGAGATCACCAAGAGGCATTCCGACCTGGAGCCATTGTGTCTCCATGGA GGCTGAAGAGTGGGGACGCCTCACTGCGGCTGCCTGGAATCCAGCTGG AGGAAGCAGGAGAGTACCGATGTGAGGTGGTGGTCACCCCTCTGAAGG CACAGGGAACAGTCCAGCTTGAAGTTGTGGCTTCCCCAGCCAGCAGAT TGTTGCTGGATCAAGTGGGCATGAAAGAGAATGAAGACAAATATATGTG TGAGTCAAGTGGGTTCTACCCAGAGGCTATTAATATAACATGGGAGAAG CAGACCCAGAAGTTTCCCCATCCCATAGAGATTTCTGAGGATGTCATCA CTGGTCCCACCATCAAGAATATGGATGGCACATTTAATGTCACTAGCTGC TTGAAGCTGAACTCCTCTCAGGAAGACCCTGGGACTGTCTACCAGTGTG TGGTACGGCATGCGTCCTTGCATACCCCCTTGAGGAGCAACTTTACCCT GACTGCTGCTCGGCACAGTCTTTCTGAAACTGAGAAGACAGATAATTTT TCCATTCATTGGTGGCCTATTTCATTCATTGGTGTTGGACTGGTTTTATTA ATTGTTTTGATTCCTTGGAAAAAGATATGTAACAAATCATCTTCAGCCTA TACTCCTCTCAAGTGCATTCTGAAACACTGGAACTCCTTTGACACTCAG ACTCTGAAGAAAGAGCACCTCATATTCTTTTGCACTCGGGCATGGCCGT CTTACCAGCTGCAGGATGGGGAGGCTTGGCCTCCTGAGGGAAGTGTTA ATATTAATACTATTCAACAACTAGATGTTTTCTGCAGACAGGAGGGCAAA TGGTCCGAGGTTCCTTATGTGCAAGCCTTCTTTGCCTTGCGAGACAACC CAGATCTTTGTCAGTGTTGTAGAATTGACCCTGCTCTCCTAACAGTTACA TCAGGCAAGTCCATAGATGATAATTCCACAAAGTCTGAGAAACAAACCC CTAGGGAACACTCGGATGCAGTTCCGGATGCCCCAATCCTTCCTGTCTC CCCTATCTGGGAACCTCCTCCAGCCACAACATCAACAACTCCAGTTCTA TCCTCCCAACCCCCAACTTTACTGTTACCCCTACAGTAA | B7H6 protein |
| 55 | GACCTGAAGGTGGAAATGATGGCAGGGGGTACTCAGATAACACCTCTC AATGATAATGTCACAATTTTTTGTAACATCTTTTACTCTCAGCCACTTAAT ATCACCTCCATGGGAATCACTTGGTTTTGGAAGTCATTGACCTTCGATAA GGAGGTGAAGGTATTTGAGTTTTTCGGGGACCACCAGGAAGCTTTCCG ACCAGGAGCAATTGTGTCCCCTTGGCGCTTGAAGTCTGGCGACGCCAG CTTGAGACTTCCTGGAATCCAATTGGAGGAAGCTGGGGAGTACAGATGT GAGGTTGTCGTGACTCCTCTGAAAGCTCAGGGTACCGTGCAGTTGGAG GTTGTCGCCTCACCCGCTTCCCGGCTTCTGCTGGACCAGGTTGGAATGA AGGAGAACGAAGATAAGTATATGTGCGAGAGTTCCGGATTTTATCCCGA GGCCATCAACATTACATGGGAGAAACAGACCCAAAAGTTTCCTCACCCC ATTGAAATCAGCGAGGATGTGATTACCGGACCCACCATTAAAAATATGG ATGGCACTTTCAACGTTACCTCATGTTTGAAATTGAACAGTAGTCAGGA AGACCCTGGAACAGTCTATCAGTGCGTCGTGAGACACGCATCCCTCCAT | Recombinant B7H6 extracellular region Fc fusion protein |
| | ACACCTCTGCGTTCAAACTTCACTCTCACTGCCGCCAGGCATAGCCTGA GCGAGACCGAGAAGACCGATAACTTCTCTCTGGAGCCTAAGAGCTGCG ACAAGACCCACACCTGTCCACCCTGTCCAGCCCCTGAGCTGCTGGGGG GGCCCTCCGTGTTCCTTTTCCCTCCAAAACCCAAGGACACCCTGATGAT CTCTAGAACCCCCGAAGTCACATGCGTGGTGGTAGATGTCTCTCATGAG GACCCCGAGGTGAAGTTCAATTGGTACGTCGATGGAGTGGAGGTGCATA ACGCCAAGACTAAGCCTAGGGAAGAGCAGTATAATTCAACCTACCGCGT CGTGTCTGTGCTCACCGTGCTGCATCAAGACTGGCTCAACGGCAAAGA ATACAAGTGCAAGGTCTCTAACAAGGCTCTGCCCGCTCCCATCGAAAAG ACCATCTCCAAGGCTAAGGGCCAACCCAGGGAGCCCCAAGTCTACACA CTGCCTCCATCACGTGAAGAGATGACTAAAAATCAAGTTAGTTTGACCT GCTTGGTTAAGGGCTTCTATCCATCTGATATAGCAGTCGAATGGGAGAGC AACGGCCAGCCTGAAAACAACTACAAGACAACTCCACCCGTCTTGGAC TCCGACGGTAGTTTTTTCCTGTACTCCAAACTGACCGTGGATAAGAGCA GATGGCAGCAGGGCAACGTGTTCTCATGTTCCGTCATGCATGAAGCTCT CCACAACCACTACACCCAGAAGTCTCTGAGTCTCTCTCCCGGTAAA | |
| 56 | GATTTGAAGGTGGAGATGATGGCTCGAGGAATTCAGGCCACCAGACTC AACGACAGCGTCACAATCTCTTGCAAAGTGATCTATAGTCAGCCCCTCA ACATAACCAGTATGGGAATTACCTGGTTTCGCAAATCACTGACTTTGGAT AAAGAGGTTAAAGTCTTTGAATTTTTTGGCGATCACCAGAAGGCCTTCA GACCCGGAGCAAATGTGTCCCTGTGGAGGCTGAAGTCTGGCGACGCTA GCCTGAAACTTCCAGGAGTTCAGCTCGAAGAGGCCGGCGAGTACAGGT GCGAAGTTGTAGTAACCCCTCTCAAAGCACAGGGCACCGTTCAGCTGA AGGTGGTGGCTTCACCTACTTCTAGACTTTTCCAGGATCAGGCCGTGGT TAAGGAGAACGAGGGCAAATATATGTGCGAGTCCTCTCGTTTTTATCCCA AAGACATCAATATCACCTGGGAGAAATGGACACAGAAATCACCTCACCA CGTTGTAATTAGCGAGAACGTGATCACAGGCCCCACAATAAAGAACATG GACGGAACTTTCAATATAACCTCCTACTTGAAGCTGAACTCCTCTCAAG AAGATCCTGGCACTGTGTATAGATGTGTGATTAGGCACGAGAGCCTCCA CACACCCGTCTCTATTGACTTCATATTGACTGCACCACAGCAGTCTCTGT CTGAACCAGAAAAGACCGATCCAAAATCCTGTGACAAGACTCATACATG TCCTCCTTGTCCAGCACCAGAGCTGCTTGGTGGCCCTAGCGTGTTCCTG TTCCCTCCCAAGCCTAAGGACACCCTGATGATTTCAAGAACCCCCGAGG TGACTTGTGTGGTCGTGGATGTGAGCCACGAGGATCCTGAAGTCAAATT CAATTGGTACGTTGATGGCGTGGAGGTGCACAATGCCAAAACCAAGCC ACGAGAGGAGCAGTACAACAGCACCTACAGAGTGGTGTCTGTGCTGAC CGTGTTGCATCAGGACTGGCTTAATGGAAAAGAATACAAGTGTAAGGTC TCCAATAAAGCTCTCCCTGCCCCAATCGAGAAGACTATCTCTAAGGCTA AAGGACAGCCAAGGGAACCCCAGGTTTACACACTGCCCCCAAGCCGAG ATGAATTGACAAAAAATCAGGTTTCCTTGACCTGCCTGGTGAAGGGTTT CTACCCATCTGATATCGCCGTTGAGTGGGAGAGCAATGGACAGCCTGAG AACAATTACAAAACAACCCCTCCTGTGTTGGATAGTGATGGCAGCTTCT TTCTGTATTCTAAGCTTACCGTGGATAAGTCTAGATGGCAGCAAGGAAAT GTGTTTTCCTGCAGCGTAATGCACGAAGCACTTCATAATCATTATACACA GAAGTCTCTCTCTCTTAGCCCAGGGAAG | Recombinant Cynomolgus monkey B7H6 extracellular region Fc fusion protein |
| 57 | ATGGCATGGAGAGCTGCATCTTCCGCTTGTGCTGCTCCTTTGCTTCTGTG GTGTGCTTTGATGACTGCCGGAGACCTGAAAGTCGAAATGATGGCTAGA GGAATCCAAGCTACTCGCCTGAATGATAGTGTGACAATCTCATGTAAAG TAATTTATTCCCAGCCTTTGAACATCACCTCTATGGGCATTACCTGGTTTC GCAAGAGTCTCACCCTGGATAAAGAGGTAAAAGTGTTCGAGTTCTTTGG AGACCATCAAAAAGCTTTCCGCCCTGGAGCTAATGTGAGCCTTTGGCGC CTGAAGAGTGGAGACGCCAGTCTGAAATTGCCTGGTGTGCAGCTCGAA GAGGCTGGTGAATATAGGTGCGAAGTGGTGGTGACTCCTCTGAAGGCTC AGGGGACCGTCCAGCTTAAAGTGGTGGCCAGTCCTACCAGTCGCCTGTT TCAGGACCAGGCAGTGGTCAAGGAGAACGAAGGTAAATATATGTGTGA | Cynomolgus monkey B7H6 protein |
| | ATCTTCTCGTTTCTACCCAAAGGATATTAATATCACCTGGGAGAAGTGGA CCCAAAAGAGCCCACACCATGTGGTCATCTCAGAGAATGTGATCACCGG CCCCACCATCAAGAATATGGATGGAACTTTCAATATCACATCTTACCTGA AGCTGAATTCAAGCCAAGAGGATCCTGGAACCGTATATAGGTGCGTCAT CAGACATGAGAGCCTCCACACACCCGTGAGCATCGACTTCATTCTGACA GCTCCACAGCAGAGTCTCAGTGAACCTGAGAAAACAGACATTTTTTCTA TCTATGGGTGGCTTTTTTCTTTTATCGCCGCTGGGCTGATCTTGCTGATCG TGCTCATCCACTGGAAAAAAGTGCCACTGCGAGAAGAAGAAGGCAGA AAC | |
| 58 | TGGGTGAGTCAACCACCTGAGATTAGAACTCTGGAGGGCAGCTCTGCAT TCTTGCCATGCTCCTTCAACGCAAGTCAGGGGAGGCTGGCTATCGGTTC CGTAACATGGTTTAGGGATGAGGTGGTGCCAGGAAAAGAAGTGAGGAA CGGAACCCCAGAGTTCAGAGGAAGACTGGCCCCTCTGGCAAGTTCCCG CTTTCTGCATGATCACCAAGCCGAACTTCACATTCGCGACGTCAGGGGG CACGACGCATCTATATACGTGTGCAGAGTGGAGGTGCTTGGTCTGGGTG TGGGCACCGGAAACGGCACTCGACTCGTCGTTGAGAAAGAGCATCCAC AGCTGGGAGAAAATCTGTACTTTCAGAGCCTCGAACCTAAGTCTTGTGA CAAGACCCATACCTGTCCTCCCTGTCCTGCTCCTGAACTGTTGGGGGGC CCCTCTGTGTTCCTGTTCCCTCCAAAGCCCAAGGACACACTGATGATTA GCAGGACACCAGAAGTCACATGTGTCGTGGTTGATGTGTCCCACGAGG ATCCCGAGGTTAAATTTAATTGGTATGTGGACGGTGTTGAGGTGCATAAC GCCAAGACCAAGCCTCGAGAAGAGCAGTACAATTCCACTTATAGGGTA GTCTCCGTATTGACCGTGCTCCATCAGGACTGGCTGAATGGCAAAGAGT ACAAATGCAAGGTCTCCAATAAGGCCCTTCCAGCCCCAATTGAAAAGAC TATTTCAAAGGCCAAGGGGCAACCCAGAGAGCCTCAGGTGTATACACTT CCTCCCTCACGTGAGGAGATGACAAAGAACCAAGTGAGTCTGACATGT CTCGTGAAAGGCTTTTACCCTTCAGACATTGCTGTGGAGTGGGAAAGCA ACGGCCAACCCGAGAACAACTACAAGACCACACCTCCCGTGCTGGACA GCGATGGGTCTTTCTTTCTCTACAGCAAGCTGACTGTGGACAAAAGTCG CTGGCAGCAAGGCAATGTCTTCAGCTGCTCTGTGATGCACGAAGCCCTC CATAATCATTATACCCAGAAGTCTCTGAGCCTGAGCCCAGGAAAG | Recombinant NKp30 extracellular region Fc fusion protein |
| 59 | CAGGTGCAGTTGCAACAATCTGGTGCAGAGCTGGTGAAACCTGGTGCA TCAGTGAAGCTTTCATGTAAAGCTTCCGGGTACACATTTACCAGCTACTA TATGTACTGGGTTAAGCAGCGTCCTGGACAGGGTCTGGAGTGGATCGGC GAGATTAATCCATCTAACGGGGGAACCAACTTTAACGAAAAGTTCAAGT CTAAGGCTACACTGACAGTCGATAAATCCTCATCCACCGCCTACATGCA GCTCTCTTCTCTGACTTCAGAGGACAGCGCCGTGTACTACTGCACTAGG ACCCTGTATTACGGTAACGACTGGTATTTCGATGTGTGGGGTGCCGGTAC AACAGTGACCGTGTCCTCAGCCAAAACAACAGCCCCTAGTGTATATCCA CTTGCCCCTGTCTGCGGCGACACTACAGGGTCTTCTGTGACACTCGGGT GTCTGGTCAAGGGGTATTTCCCTGAGCCAGTGACCCTCACTTGGAACTC AGGCTCCCTTTCTAGCGGCGTCCACACATTTCCAGCCGTGCTTCAGTCT GATCTGTATACCCTGAGCAGCAGCGTGACCGTCACCTCCTCCACATGGC CTTCACAAAGCATAACTTGTAATGTCGCCCACCCTGCCTCATCTACCAAA GTCGACAAGAAGATCGAGCCTCGAGGCCCCACTATCAAACCCTGTCCA CCCTGTAAGTGCCCCGCTCCAAACTTGCTGGGCGGACCATCCGTTTTCA TCTTCCCTCCAAAGATCAAAGACGTGCTGATGATTTCACTCAGCCCTATT GTCACTTGTGTAGTGGTGGACGTGTCAGAAGATGATCCTGACGTGCAGA TTTCCTGGTTTGTCAACAATGTTGAGGTGCATACAGCTCAGACACAGAC CCACCGCGAAGATTATAACTCTACTTTGAGGGTCGTTTCCGCCCTGCCA ATTCAGCACCAGGACTGGATGTCTGGAAAGGAATTCAAATGTAAAGTGA ATAATAAGGACCTCCCCGCTCCCATCGAACGGACTATCTCTAAGCCTAAA GGGAGCGTACGAGCTCCTCAGGTGTATGTCCTGCCCCCCCCAGAGGAA GAAATGACCAAGAAACAGGTGACCCTTACCTGTATGGTTACTGACTTTA TGCCTGAGGACATCTATGTAGAGTGGACCAACAACGGGAAGACTGAGT | Murine 12F5 heavy chain variable region |
| | TGAACTACAAAAACACAGAACCTGTGCTTGATAGTGATGGTTCTTATTT CATGTATAGCAAGCTCAGAGTCGAAAAAAAAAACTGGGTGGAACGCAA TTCTTACTCCTGTTCCGTGGTGCACGAGGGGTTGCACAATCACCACACT ACAAAAAGCTTCAGTAGGACCCCCGGCAAG | |
| 60 | CAAATCGTGCTGACCCAGTCTCCAGCAATAATGTCCGCATCTCCTGGTG AGAAAGTGACCATGACTTGTTCAGCTTCCTCTTCTGTTAACTATATGCAC TGGTACCAACAGAAGTCTGGCACCTCCCCAAAGCGGTGGATTTATGACA CCTCCAAGCTGGCTAGCGGGGTACCCGCAAGGTTCTCCGGTTCAGGCA GCGGCACTTCTTACTCCCTGACTATTAGCTCTATGGAGGCAGAGGATGCC GCTACCTACTATTGCCAGCAATGGAGCAGTAATCCAGTCACATTTGGGG CCGGAACCAAGCTGGAGCTGAAGCGAGCTGATGCAGCTCCCACAGTAT CTATTTTTCCTCCATCTTCTGAGCAGCTCACATCTGGTGGGGCCTCCGTG GTCTGCTTCTTGAATAACTTCTATCCTAAGGACATTAACGTGAAATGGAA GATCGACGGATCCGAACGGCAGAACGGAGTGCTTAACAGCTGGACCGA CCAGGATAGTAAGGATTCTACATATTCCATGTCTTCCACCCTCACACTCA CAAAGGACGAGTATGAGAGACACAACAGCTACACATGTGAAGCCACTC ACAAAACCTCCACATCTCCAATTGTGAAGTCCTTTAATAGGAATGAGTG T | Murine 12F5 light chain variable region |
| 61 | GAAGTTCTGCTTCAGCAGTCTGGCCCAGAAGTTGTTAAGCCAGGAGCC TCCGTGAAAATTACTTGCAAGGCCTCCGGGTACACATTTACAGACTACA ATATGGACTGGGTGAAGCAGTCCCATGGAAAGAGTCTGGAATGGATCGG CGATATCAATCCAAACAATGGCGGTACCCTCTATAACCAGAAATTTCGCG GACGTGTGACCTTGATCGTCGACAAAAGTAGTTCTACTGCCTACATGGA GCTGCGCAGCCTGACTAGTGACGACACCGCCGTCTACTATTGCGCTCGC TCTGAAGTCTTTTATGGGAATTATGCTGACTACTGGGGCCAGGGTACCAC TTTGACCGTCTCCAGT GCCAAAACAACAGCCCCTAGTGTATATCCACTTGCCCCTGTCTGCGGCG ACACTACAGGGTCTTCTGTGACACTCGGGTGTCTGGTCAAGGGGTATTT CCCTGAGCCAGTGACCCTCACTTGGAACTCAGGCTCCCTTTCTAGCGGC GTCCACACATTTCCAGCCGTGCTTCAGTCTGATCTGTATACCCTGAGCAG CAGCGTGACCGTCACCTCCTCCACATGGCCTTCACAAAGCATAACTTGT AATGTCGCCCACCCTGCCTCATCTACCAAAGTCGACAAGAAGATCGAGC CTCGAGGCCCCACTATCAAACCCTGTCCACCCTGTAAGTGCCCCGCTCC AAACTTGCTGGGCGGACCATCCGTTTTCATCTTCCCTCCAAAGATCAAA GACGTGCTGATGATTTCACTCAGCCCTATTGTCACTTGTGTAGTGGTGGA CGTGTCAGAAGATGATCCTGACGTGCAGATTTCCTGGTTTGTCAACAAT GTTGAGGTGCATACAGCTCAGACACAGACCCACCGCGAAGATTATAACT CTACTTTGAGGGTCGTTTCCGCCCTGCCAATTCAGCACCAGGACTGGAT GTCTGGAAAGGAATTCAAATGTAAAGTGAATAATAAGGACCTCCCCGCT CCCATCGAACGGACTATCTCTAAGCCTAAAGGGAGCGTACGAGCTCCTC AGGTGTATGTCCTGCCCCCCCCAGAGGAAGAAATGACCAAGAAACAGG TGACCCTTACCTGTATGGTTACTGACTTTATGCCTGAGGACATCTATGTA GAGTGGACCAACAACGGGAAGACTGAGTTGAACTACAAAAACACAGA ACCTGTGCTTGATAGTGATGGTTCTTATTTCATGTATAGCAAGCTCAGAG TCGAAAAAAAAAACTGGGTGGAACGCAATTCTTACTCCTGTTCCGTGGT GCACGAGGGGTTGCACAATCACCACACTACAAAAAGCTTCAGTAGGAC CCCCGGCAAG | Murine 12G4 heavy chain variable region |
| 62 | GACATTGTGCTGACTCAGAGTCCTGTAAGTCTGGCAGTTCCCCTCGGCC AAAGAGCTACCATTAGTTGTAAGGCTTCCCAGAGCGTGGACTATGATGG CGACAGCTATATGAATTGGTATCAACAGAAGCCAGGACAGCCACCCAAG CTGCTGATCTACGCCGCCTCCACCCTGCATTCAGGTATCCCTGTGCGATT TTCTGGTAGTGGCTCCGGCACAGATTTCACCCTGAATATCCATCCAGTTG AGGAGGAGGATGCTGCCTCTTACTACTGTCAGCAGAGCAAAGAGGATC CTAGAACCTTCGGCGGTGGCACTAAGCTGGAAATTAAGCGAGCTGATGC AGCTCCCACAGTATCTATTTTTCCTCCATCTTCTGAGCAGCTCACATCTG GTGGGGCCTCCGTGGTCTGCTTCTTGAATAACTTCTATCCTAAGGACATT AACGTGAAATGGAAGATCGACGGATCCGAACGGCAGAACGGAGTGCTT | Murine 12G4 light chain variable region |
| | AACAGCTGGACCGACCAGGATAGTAAGGATTCTACATATTCCATGTCTTC CACCCTCACACTCACAAAGGACGAGTATGAGAGACACAACAGCTACAC ATGTGAAGCCACTCACAAAACCTCCACATCTCCAATTGTGAAGTCCTTT AATAGGAATGAGTGT | |
| 63 | GAAGTTCAACTGGTTCAGAGCGGAGCTGAGGTAAAGAAGCCAGGTGCC AGCGTGAAAGTTAGTTGCAAGGCATCCGGATACACTTTCACTAGCTATT ATATGTATTGGGTGCGACAGGCCCCAGGGCAAGGTTTGGAGTGGATCGG AGAGATCAATCCTTCAAATGGCGGCACAAATTTCAACGAAAAGTTCAA ATCTCGCGTGACTCTGACAGTGGACAAGTCCATCTCCACAGCATATATG GAGCTGAGCCGGCTTAGATCCGATGACACTGCTGTCTACTATTGTACAA GAACACTGTACTATGGCAACGACTGGTACTTCGATGTCTGGGGACAGGG GACCACTGTGACCGTCTCCAGTGCCAGTACAAAGGGACCATCTGTGTTT CCTCTGGCACCTTCATCTAAGAGCACTTCTGGTGGTACTGCTGCTCTGG GCTGCCTTGTAAAGGACTATTTCCCAGAGCCCGTGACCGTCAGTTGGAA TTCAGGCGCCCTTACCAGCGGGGTTCACACTTTCCCAGCTGTGTTGCAG TCTTCAGGGCTTTACAGTCTCAGCAGTGTTGTCACCGTCCCTTCCTCCTC ACTGGGCACCCAAACCTATATTTGTAATGTCAACCATAAACCAAGTAAC ACCAAGGTGGACAAGAAGGTGGAGCCCAAGTCATGTGATAAAACTCAC ACTTGTCCACCATGCCCTGCTCCCGAACTGTTGGGCGGACCTAGCGTGT TTCTCTTTCCACCCAAACCAAAGGATACCCTGATGATCAGCAGGACTCC CGAGGTGACTTGTGTGGTGGTTGACGTGTCCCATGAGGATCCTGAAGTG AAGTTCAATTGGTACGTGGACGGCGTGGAGGTTCATAACGCAAAGACC AAGCCCAGGGAAGAGCAGTATAACTCTACTTACCGGGTGGTTTCAGTCT TGACTGTGTTGCACCAGGACTGGCTGAATGGAAAGGAGTATAAGTGTA AAGTGAGTAACAAAGCCCTGCCAGCTCCCATTGAGAAGACAATCTCAA AAGCTAAGGGTCAGCCCCGCGAACCACAGGTGTATACACTCCCACCCTC CCGCGAGGAGATGACCAAAAACCAGGTGTCCCTCACCTGCCTGGTCAA GGGATTTTACCCCAGCGATATTGCTGTCGAGTGGGAGTCAAACGGTCAG CCCGAGAATAATTATAAAACTACCCCTCCCGTGCTTGACAGCGATGGATC TTTCTTCCTGTACTCAAAACTGACCGTCGACAAATCCCGATGGCAGCAA GGTAACGTGTTCTCTTGCAGCGTAATGCACGAGGCTCTTCACAATCACT ATACTCAGAAGTCACTCAGCCTTAGTCCTGGAAAG | Humanized 12F5 heavy chain variable region |
| 64 | GAAATCGTGCTGACCCAGAGCCCAGATTTCCAGAGTGTCACACCAAAG GAGAAGGTAACAATTACATGCAGTGCCAGCAGTAGCGTGAACTATATGC ATTGGTATCAGCAGAAACCCGATCAGTCCCCTAAGCGACTGATCTACGA TACCTCCAAGCTGGCCAGCGGTGTTCCAAGTAGGTTCTCTGGCAGCGGG TCTGGGACAGACTACACTCTCACTATCAACTCTCTGGAAGCTGAGGATG CTGCTACTTATTATTGCCAGCAGTGGAGCTCAAATCCCGTTACCTTTGGC CAGGGTACCAAGCTCGAAATTAAACGTACAGTCGCAGCACCTAGTGTCT TCATCTTTCCTCCCTCTGACGAGCAGTTGAAGAGCGGAACTGCAAGCGT CGTTTGCCTCCTGAACAATTTCTATCCCCGGGAGGCTAAGGTCCAGTGG AAAGTGGATAATGCTCTCCAGAGCGGTAACTCACAAGAGTCTGTTACCG AGCAGGACTCCAAGGATTCTACCTATAGTCTGAGCTCTACCCTGACACT GTCCAAGGCCGATTACGAGAAACACAAAGTGTATGCCTGCGAAGTAAC CCACCAGGGCCTGAGTTCCCCAGTGACCAAGTCTTTCAATAGGGGGGA GTGT | Humanized 12F5 light chain variable region |
| 65 | GAAGTGCAGCTTGTACAGAGTGGAGCAGAGGTCAAGAAACCAGGAGC TTCCGTGAAGGTGAGCTGCAAGGCCTCCGGCTATACCTTTACTGATTAC AACATGGACTGGGTCCGTCAGGCTCCTGGAAAAGGACTGGAGTGGATT GGTGACATTAATCCTAATAACGGCGGCACACTTTACAATCAGAAGTTTC GTGGTAGAGTTACACTGACTGTGGATAAATCAATCAGCACCGCCTATATG GAGCTGTCCCGACTGCGGTCCGACGATACTGCAGTGTATTACTGCGCCA GGTCAGAGGTGTTTTACGGTAACTACGCAGACTATTGGGGCCAGGGGAC AACAGTGACAGTCAGCAGCGCCAGTACAAAGGGACCATCTGTGTTTCC TCTGGCACCTTCATCTAAGAGCACTTCTGGTGGTACTGCTGCTCTGGGC TGCCTTGTAAAGGACTATTTCCCAGAGCCCGTGACCGTCAGTTGGAATT CAGGCGCCCTTACCAGCGGGGTTCACACTTTCCCAGCTGTGTTGCAGTC | Humanized 12G4 heavy chain variable region |
| | TTCAGGGCTTTACAGTCTCAGCAGTGTTGTCACCGTCCCTTCCTCCTCAC TGGGCACCCAAACCTATATTTGTAATGTCAACCATAAACCAAGTAACAC CAAGGTGGACAAGAAGGTGGAGCCCAAGTCATGTGATAAAACTCACAC TTGTCCACCATGCCCTGCTCCCGAACTGTTGGGCGGACCTAGCGTGTTT CTCTTTCCACCCAAACCAAAGGATACCCTGATGATCAGCAGGACTCCCG AGGTGACTTGTGTGGTGGTTGACGTGTCCCATGAGGATCCTGAAGTGAA GTTCAATTGGTACGTGGACGGCGTGGAGGTTCATAACGCAAAGACCAA GCCCAGGGAAGAGCAGTATAACTCTACTTACCGGGTGGTTTCAGTCTTG ACTGTGTTGCACCAGGACTGGCTGAATGGAAAGGAGTATAAGTGTAAA GTGAGTAACAAAGCCCTGCCAGCTCCCATTGAGAAGACAATCTCAAAA GCTAAGGGTCAGCCCCGCGAACCACAGGTGTATACACTCCCACCCTCCC GCGAGGAGATGACCAAAAACCAGGTGTCCCTCACCTGCCTGGTCAAGG GATTTTACCCCAGCGATATTGCTGTCGAGTGGGAGTCAAACGGTCAGCC CGAGAATAATTATAAAACTACCCCTCCCGTGCTTGACAGCGATGGATCTT TCTTCCTGTACTCAAAACTGACCGTCGACAAATCCCGATGGCAGCAAGG TAACGTGTTCTCTTGCAGCGTAATGCACGAGGCTCTTCACAATCACTATA CTCAGAAGTCACTCAGCCTTAGTCCTGGAAAG | |
| 66 | GACATAGTATTGACCCAGACACCATTGTCTTTGAGCGTTACCCCAGGCC AGCCAGCTTCAATATCTTGCAAGGCCTCTCAGTCCGTCGACTATGATGGC GATTCCTACATGAATTGGTACCTCCAAAAGCCAGGCCAACCACCACAGC TGTTGATTTATGCCGCTTCTACACTCCACAGTGGCGTGCCCGATCGATTT TCAGGATCCGGCTCAGGGACCGATTTCACCCTTAAGATTTCTCGAGTGG AAGCCGAGGATGTGGGCGTGTATTACTGCCAGCAGAGCAAAGAGGATC CTAGAACTTTTGGCCAGGGAACCAAGCTGGAGATTAAACGTACAGTCG CAGCACCTAGTGTCTTCATCTTTCCTCCCTCTGACGAGCAGTTGAAGAG CGGAACTGCAAGCGTCGTTTGCCTCCTGAACAATTTCTATCCCCGGGAG GCTAAGGTCCAGTGGAAAGTGGATAATGCTCTCCAGAGCGGTAACTCAC AAGAGTCTGTTACCGAGCAGGACTCCAAGGATTCTACCTATAGTCTGAG CTCTACCCTGACACTGTCCAAGGCCGATTACGAGAAACACAAAGTGTAT GCCTGCGAAGTAACCCACCAGGGCCTGAGTTCCCCAGTGACCAAGTCT TTCAATAGGGGGGAGTGT | Humanized 12G4 light chain variable region |

Hereinafter, the present disclosure will be described in detail by way of examples. In the examples or test examples, experimental methods without specifying the specific conditions were carried out according to conventional conditions.

Hereinafter, the scheme of the present disclosure will be explained in conjunction with examples. Those skilled in the art will understand that the following examples are only used to illustrate the present disclosure and should not be considered as limiting the scope of the present disclosure. Where specific techniques or conditions are not specified in the examples, they are performed according to techniques or conditions described in the literature in the art or according to the product specification. The reagents or instruments used without specifying the manufacturer are all conventional products that can be obtained commercially.

### Examples 1 Preparation of antibody

Balb/c mice (9-week-old, purchased from Shanghai SLAC, weighing about 20 g) were immunized with purified recombinant B7H6 extracellular region Fc fusion protein (B7H6-Fc) (recombinant B7H6 extracellular region Fc fusion protein, amino acid sequence as set forth in SEQ ID NO. 22) as an antigen to generat murine monoclonal antibody against human B7H6.

Mice were immunized three times with purified antigen and a complete Freund's adjuvant, and immune responses were detected after exsanguination via the tail vein. Sera were screened by ELISA and flow cytometry to obtain mice with anti-human B7H6 immunoglobulin. Splenocytes from mice with the highest anti-B7H6 immunoglobulin were taken out and fused with murine myeloma SP2/0 cells (ATCC NO. CRL-1581). The fused hybridoma cells were screened for antibodies to obtain murine mAbs.

The candidate hybridoma cells were cultured to a total number of 10⁶. The cells were collected by centrifugation at 800 rpm for 10 min, and the total RNA was extracted with a Trizol kit (Invitrogen). The total RNA was used as a template to synthesize the cDNA library (Invitrogen) by reverse transcription, and cDNA was used as a template to amplify the corresponding nucleic acid sequence of the variable region in hybridoma cells by PCR. The primer sequences used in the PCR amplification reaction were complementary to first framework region of the antibody variable region or signal peptide region and the constant region (Larrick, J.W., et al., (1990) Scand. J. Immunol., 32, 121-128 and Coloma, J.J. et al., (1991) BioTechniques, 11, 152-156). In a 50 µL reaction system, 2 µL of cDNA, 5 µL of 10 × PCR buffer, 2 µL of upstream and downstream primers (5 µmol), 2 µL of dNTP, 1 µL of Taq enzyme (Takara, Ex Taq), and 38 µL of H₂O were added respectively. A pre-denaturation was performed for 5 min at 95°C, and the procedure was entered into the temperature cycle for PCR amplification. The reaction conditions included: denaturation at 94°C for 30 s, annealing at 58°C for 45 s, extension at 72°C for 50 s for 32 cycles, then extension at 72°C for 7 min. After sequencing the amplification products, the heavy and light chain variable region sequences (including amino acid sequences and nucleic acid sequences) of murine mAb were obtained.

### Example 2: ELISA binding assay of murine B7H6 antibody

The ELISA assay was used to detect the binding characteristics of the B7H6 antibody. The B7H6 extracellular region Fc fusion protein (B7H6-Fc) described above was coated into a 96-well plate. The signal strength after antibody addition was used to determine the binding characteristic of the antibody and B7H6.

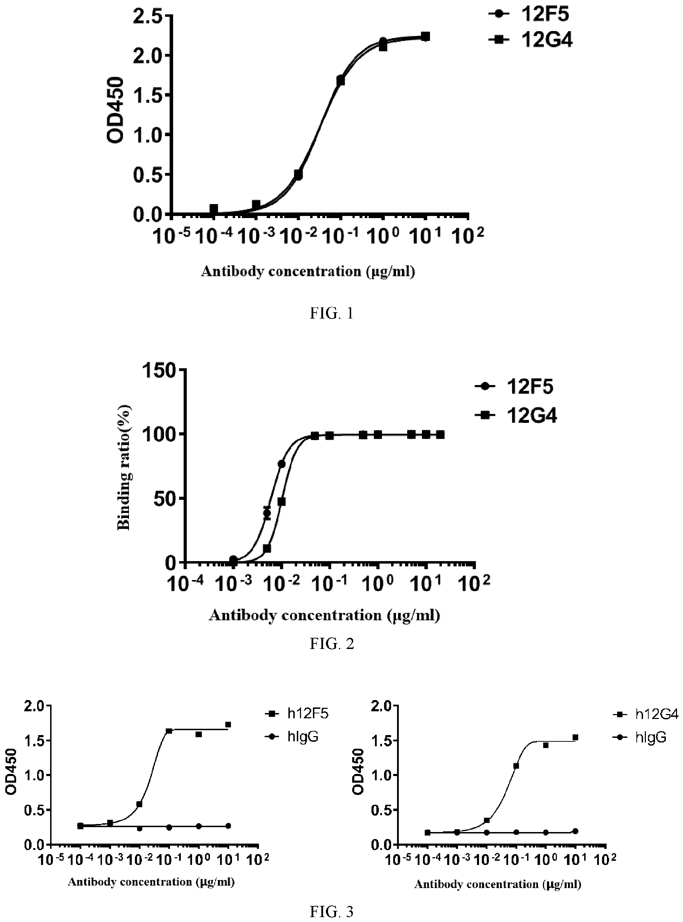

The B7H6-Fc fusion protein (amino acid sequence as set forth in SEQ ID NO. 22) was diluted to 1 µg/mL with PBS buffer and added to a 96-well plate in a volume of 100 µL/well and left overnight at 4°C. The PBS buffer solution was sucked off from the 96-well plate. The plate was washed 6 times with PBST (pH 7.2, PBS containing 0.1% Tween 20) buffer solution. 200 µL/well PBS/10% BSA was added and incubated at 37°C for 2 hours for blocking. The blocking solution was removed. The plate was washed 6 times with PBST. 100 µL/well B7H6 antibody to be tested was diluted to an appropriate concentration with PBST/0.05% BSA, and incubated at 37°C for 1 h. The reaction system was removed. The plate was washed 6 times with PBST. HRP (horseradish peroxidase)-labeled anti-mouse antibody secondary antibody was diluted with PBST/0.05% BSA at 100 µL/well and incubated for 1 h at 37°C. The plate was washed 6 times with PBST and 80 µL/well TMB (tetramethylbenzidine) was added. After 3 min of incubation at room temperature, 80 µL/well 4M sulfuric acid was added to terminate the reaction. The absorbance at 450 mm was read with a microplate reader. The results (FIG. 1) show that murine antibodies 12F5 (SEQ ID NO. 26 and SEQ ID NO. 27) and 12G4 (SEQ ID NO. 28 and SEQ ID NO. 29) of the present disclosure are capable of binding to B7H6.

### Example 3: Flow cytometry assay for binding of Murine B7H6 antibody

The flow cytometry assay was used to detect the binding characteristics of B7H6 antibody and to overexpress B7H6 protein (HL60-B7H6, SEQ ID NO.21; SEQ ID NO.54) in HL60 cells (ATCC NO. CCL-240). The expression vector is pLUX-EF1a-B7H6-IRES-ZsGreen, and the signal strength after antibody addition is used to determine the binding characteristics of the antibody and B7H6.

HEK293T cells were plated in a six-well plate at 5×10⁵ cells/well and cultured overnight in a DMEM medium without diabody. The medium was discarded before transfection and 1 mL of fresh DMEM medium without diabody was added. The pLVX-EF1a-B7H6-IRES-ZsGreen (the encoding sequence (SEQ ID NO. 54) of B7H6 protein (SEQ ID NO: 21) was inserted between the restriction endonuclease sites EcoRI and BamHI of the pLVX-EF1a-IRES-ZsGreen1 vector), pMD2G, psPAX2 vector (a total of 3 µg) were added in a ratio of 2: 1: 1 to 200 µl of serum-free DMEM medium, and 12 µg of polyetherimide (PEI, polysciences Co. Ltd.) was added. After mixing well, the mixture was stood for 16 min. Then the whole liquid was added to a six-well plate plated with HEK293T cells. After 6 h of culture, the medium was discarded and a fresh complete DMEM medium was added for culture. After 48 h of transfection, the cell culture supernatant was collected and passed through a 0.45 µm filter (Millipore) to obtain the virus supernatant. All viral supernatant was added to a 6-well plate containing 1 × 10⁴ HL60 cells, added with polybrene (Sigma) at a final concentration of 4 µg/ ml, and incubated for 12 h. The supernatant was then discarded and a fresh complete RPMI1640 medium was added. The resulting cells were HL60-B7H6 cells.

HL60-B7H6 cells were diluted to 2 × 10⁶/mL with PBS and were added to a 1.5 ml EP tube at 100 µL/tube. Goat serum at 10 µL/tube was added thereto and was blocked at 4°C for 30 min. The B7H6 antibody was added at 1 µg/tube and incubated for 30 min at 4°C. The EP tube was added with 1 mL of PBS and centrifuged at 3500 rpm at 4°C for 5 min. The supernatant was discarded and the precipitate was washed once more with PBS. After centrifugation, the supernatant was discarded, and the cells were resuspended with 100 µl/tube of PBS, and 0.1 µL/tube of goat anti-mouse antibody secondary antibody (Invitrogen) labeled with Alexa-647 was added thereto, and incubated at 4°C, under the condition of avoiding light, for 30 min. The cell-containing precipitate was washed twice with PBS and the supernatant was discarded after centrifugation. The cells were resuspended with 200 µL/tube of PBS and detected by flow cytometry. The result is as shown in FIG. 2, further showing that the murine antibodies 12F5 (SEQ ID NO. 26 and SEQ ID NO. 27), 12G4 (SEQ ID NO. 28 and SEQ ID NO. 29) of the present disclosure can bind B7H6.

### Example 4: Mouse antibody humanization experiment

With reference to the sequence of the light chain variable region and the sequence of the heavy chain variable region of the B7H6 antibody, the humanized template that best matches the non-CDR region thereof is selected. The CDR region of the murine antibody was transplanted onto the selected humanized template to replace the CDR region of humanized template to obtain a humanized antibody. Then, based on the three-dimensional structure of the murine antibody, reverse mutations were performed on the embedded residues, the residues that have direct interaction with the CDR region, and the residues that have important influence on the conformation of VL and VH to obtain a humanized antibody. The sequence of the heavy chain variable region of 12F5 of the humanized B7H6 antibody is as set forth in SEQ ID NO. 17 and the sequence of the light chain variable region is as set forth in SEQ ID NO. 18, or the sequence of the heavy chain variable region of 12G4 of the humanized B7H6 antibody is as set forth in SEQ ID NO. 19 and the sequence of the light chain variable region is as set forth in SEQ ID NO. 20.

### Example 5: ELISA binding assay of humanized B7H6 antibody

The ELISA assay was used to detect the binding characteristics of the humanized B7H6 antibody. The B7H6 extracellular region Fc fusion protein (B7H6-Fc) described above was coated into a 96-well plate. The signal strength after antibody addition was used to determine the binding characteristics of antibodies and B7H6.

The B7H6-Fc fusion protein (amino acid sequence as set forth in SEQ ID NO. 22) was diluted to 1 µg/mL with PBS buffer and added to a 96-well plate in a volume of 100 µL/well and left overnight at 4°C. The PBS buffer solution was sucked off from the 96-well plate. The plate was washed 6 times with PBST (pH 7.2, PBS containing 0.1% Tween 20) buffer solution. 200 µL/well PBS/10% BSA was added and incubated at 37°C for 2 hours for blocking. The blocking solution was removed. The plate was washed 6 times with PBST. B7H6 antibody to be tested which diluted with PBST/0.05% BSA to an appropriate concentration was added at 100 µL/well, and incubated at 37°C for 1 h. The reaction system was removed. The plate was washed 6 times with PBST. HRP (horseradish peroxidase)-labeled mouse anti-human IgG (Fab-specific) secondary antibody (Sigma) was diluted with PBST/0.05% BSA at 100 µL/well and incubated for 1 h at 37°C. The plate was washed 6 times with PBST, and 80 µL/well TMB (tetramethylbenzidine) was added. After 3 min of incubation at room temperature, and 80 µL/well 4M sulfuric acid was added to terminate the reaction. The absorbance at 450 mm was read with a microplate reader. The results (FIG. 3) show that humanized antibodies h12F5 (SEQ ID NO. 30 and SEQ ID NO. 31), h12G4 (SEQ ID NO. 32 and SEQ ID NO. 33) of the present disclosure can bind B7H6.

### Example 6: Flow cytometry assay for binding of Humanized B7H6 antibody

The flow cytometry assay was used to detect the binding characteristics of the B7H6 antibody. The B7H6 protein (HL60-B7H6) was overexpressed in HL60 cells (ATCC No. CCL-240) (as shown in Example 3), and the signal strength after antibody addition was used to determine the binding characteristics of the antibody and B7H6.

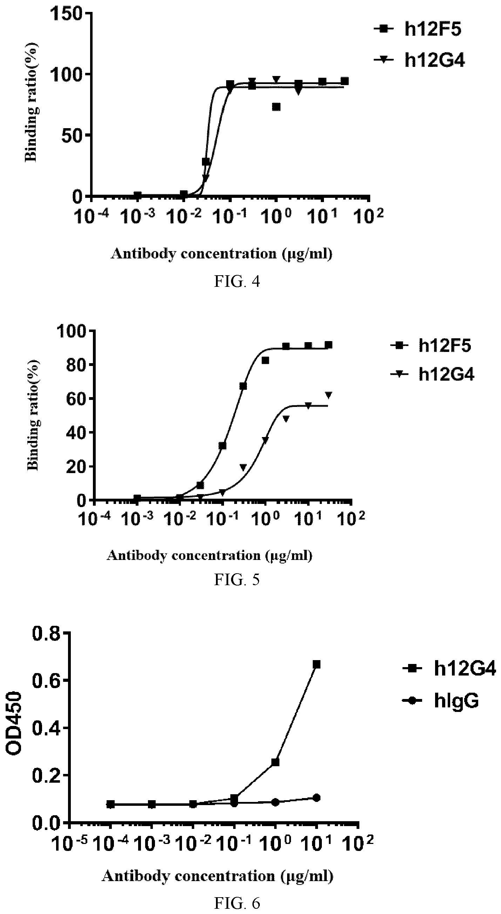

HL60-B7H6 cells were diluted to 2 × 10⁶/mL with PBS and added to a 1.5 mL EP tube in a volume of 100 µL/tube. 10 µL/tube of mouse serum was added thereto and was blocked at 4°C for 30 min. The humanized B7H6 antibody was added at 1 µg/tube and incubated for 30 min at 4°C. The EP tube was added with 1 mL of PBS and centrifuged at 3500 rpm at 4°C for 5 min. The supernatant was discarded and the precipitate was washed once more with PBS. After centrifugation, the supernatant was discarded. The cells were resuspended with 100 µL/tube of PBS, 1 µL/tube of Alexa-647-labeled mouse anti-human IgG-Fc secondary antibody (Biolegend) was added thereto, and incubated at 4°C, under the condition of avoiding light, for 30 min. The cell-containing precipitate was washed twice with PBS and the supernatant was discarded after centrifugation. The cells were resuspended with 200 µL/tube of PBS and detected by flow cytometry. The result is as shown in FIG. 4, further showing that humanized antibodies h12F5 (SEQ ID NO. 30 and SEQ ID NO. 31), h12G4 (SEQ ID NO. 32 and SEQ ID NO. 33) of the present disclosure can bind to B7H6.

### Example 7: Flow cytometry assay for binding of humanized B7H6 antibody

The flow cytometry assay was used to detect the binding characteristics of the humanized B7H6 antibody. Cynomolgus monkey B7H6 protein (CHO-K1-cyno-B7H6) was overexpressed in CHO-K1 cells (ATCC No. CCL-240). The signal strength after antibody addition was used to determine the binding characteristics of the antibody and Cynomolgus monkey B7H6.

HEK293T cells were plated in a six-well plate at 5 × 10⁵ cells/well and cultured overnight in a DMEM medium without diabody. The medium was discarded before transfection and 1 mL of fresh DMEM medium without diabody was added. The pLVX-EF1a-cynoB7H6-IRES-ZsGreen (the encoding sequence (SEQ ID NO. 57) of monkey B7H6 protein (SEQ ID NO: 24) was inserted between the restriction endonuclease sites EcoRI and BamHI of the pLVX-EF1a-IRES-ZsGreen1 vector), pMD2G, psPAX2 vector (a total of 3 µg) were added in a ratio of 2: 1: 1 to 200 µL of a serum-free DMEM medium, and 12 µg of polyetherimide (PEI, polysciences Co. Ltd.) was added. After mixing well, the mixture was stood for 16 min. Then the whole liquid was added to a six-well plate plated with HEK293T cells. After 6 h of culture, the medium was discarded and a fresh complete DMEM medium was added for culture. After 48 h of transfection, the cell culture supernatant was collected and passed through a 0.45 µm filter (Millipore) to obtain the virus supernatant. All viral supernatant was added to a 6-well plate containing 1 × 10⁴ CHO-K1 cells, polybrene (Sigma) at a final concentration of 4 µg/mL was added, and cultured for 12 h. The supernatant was then discarded and a fresh complete RPMI1640 medium was added. The resulting cells were CHO-K1-cyno B7H6 cells.

CHO-K1-cynoB7H6 cells were diluted to 2 × 10⁶/mL with PBS and added to a 1.5 ml EP tube in a volume of 100 µL/tube. 10 µL/tube of mouse serum was added thereto and was blocked at 4°C for 30 min. 1 µg/tube of the humanized B7H6 antibody was added and incubated for 30 min at 4°C. The EP tube was added with 1 mL of PBS and centrifuged at 3500 rpm at 4°C for 5 min. The supernatant was discarded and the precipitate was washed once more with PBS. After centrifugation, the supernatant was discarded. The cells were resuspended with 100 µL/tube of PBS, 1 µL/tube of Alexa-647-labeled mouse anti-human IgG-Fc secondary antibody (Biolegend) was added thereto, and incubated at 4°C, protected from light, for 30 min. The cell-containing precipitate was washed twice with PBS and the supernatant was discarded after centrifugation. The cells were resuspended with 200 µL/tube of PBS and detected by flow cytometry. The result is shown in FIG. 5, further showing that the antibodies h12F5, h12G4 of the present disclosure can bind to monkey B7H6.

### Example 8: ELISA assay for binding of humanized B7H6 antibody

The ELISA assay was used to detect the binding characteristics of the humanized B7H6 antibody. The Cynomolgus monkey B7H6 extracellular Fc fusion protein (cynoB7H6-Fc) was coated into a 96-well plate. The signal strength after antibody addition was used to determine the binding characteristics of the antibody and monkey B7H6.

The cynoB7H6-Fc fusion protein (amino acid sequence as set forth in SEQ ID NO. 23) was diluted to 1 µg/mL with PBS buffer and added to a 96-well plate in a volume of 100 µL/well and left overnight at 4°C. The PBS buffer solution was sucked off from the 96-well plate. The plate was washed 6 times with PBST (pH 7.2, PBS containing 0.1% Tween 20) buffer solution. 200 µL/well of PBS/10% BSA was added and incubated at 37°C for 2 hours for blocking. The blocking solution was removed. The plate was washed 6 times with PBST. B7H6 antibody to be tested which was diluted with PBST/0.05% BSAto an appropriate concentration was added at 100 µL/well, and incubated at 37°C for 1 h. The reaction system was removed. The plate was washed 6 times with PBST. HRP (horseradish peroxidase)-labeled mouse anti-human IgG (Fab-specific) secondary antibody (Sigma) was diluted with PBST/0.05% BSA at 100 µL/well and incubated for 1 h at 37°C. The plate was washed 6 times with PBST and 80 µL/well of TMB (tetramethylbenzidine) was added. After 3 min of incubation at room temperature, 80 µL/well of 4M sulfuric acid was added to terminate the reaction. The absorbance at 450 mm was read with a microplate reader. The results (FIG. 6) show that humanized antibody h12G4 of the present disclosure can bind to monkey B7H6.

### Example 9: In vitro binding affinity and kinetic experiments

The Biacore method is widely-recognized as an objective method for detecting the mutual affinity and binding kinetics of proteins. The affinity and binding kinetics of the B7H6 antibody of the present disclosure are analyzed and characterized through Biacore T200.

The B7H6 extracellular segment Fc fusion protein (B7H6-Fc) was covalently attached to a CM5 (GE) chip using standard amino coupling methods. A series of concentration gradients of B7H6 antibody diluted in PBS were then injected with each cycle and regenerated with 10 mM NaOH solution. The antigen-antibody binding kinetics were traced for 3 minutes and the dissociation kinetics were traced for 10 minutes, and the resulting data were analyzed by a 1: 1 (Langmuir) binding model using GE's BIAevaluation software. The KD values determined in this way were 2.2 nM for murine 12F5 and 0.15nM for murine 12G4.

### Example 10: Screening for the ability of the antibody to promote the binding of B7H6 to NKp30

The B7H6 antibody can promote the signaling pathway of B7H6 and its receptor NKp30 by binding to the extracellular domain of B7H6. The flow cytometry assay was used to detect the enhancement of B7H6 antibody binding to the receptor NKp30.

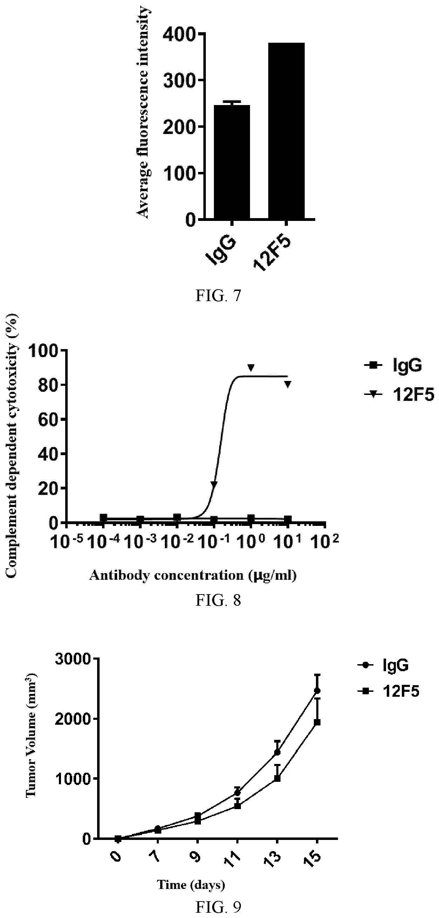

HL60-B7H6 cells (same as above) were diluted with PBS to 2 × 10⁶/ml, added to a 1.5 mL EP tube in a volume of 100 µL/tube, 10 µL/tube of mouse serum was added thereto, and was blocked at 4°C for 30 min. NKp30 extracellular region Fc fusion protein (NKp30-Fc, SEQ ID NO. 25, expressed in this laboratory) was added at 4 µg/tube and incubated for 30 min at 4°C. B7H6 antibody was added and incubated at 4°C for 30 min. The EP tube was added with 1 mL of PBS and centrifuged at 3500 rpm at 4°C for 5 min. The supernatant was discarded and the precipitate was washed once more with PBS. After centrifugation, the supernatant was discarded. The cells were resuspended with 100 µl/tube of PBS, 1 µL/tube of647-labeled mouse anti-human antibody secondary antibody (Biolegend) was added thereto, and incubated at 4°C, under the condition of avoiding light, for 30 min. The cell-containing precipitate was washed twice with PBS and the supernatant was discarded after centrifugation. The cells were resuspended with 200 µL/tube of PBS and detected by flow cytometry. The results are shown in FIG. 7. It can be seen that the murine 12F5 antibody of the present disclosure can promote the binding of B7H6 to the receptor NKp30.

### Example 11: complement dependent cytotoxicity detection

Complement dependent cytotoxicity assay was used to detect the killing effect of B7H6 antibody on tumor cells. Among them, the tumor cells used were HL60-B7H6 cells.
(1) Tumor cells were resuspended with a serum-free RPMI-1640 medium and counted. The cells were diluted to 4 × 10⁵/mL with a complete RPMI-1640 medium. The diluted cells were added to a 96-well round bottom plate in a volume of 25 µL/well.
(2) The B7H6 antibody or control murine IgG was diluted with a serum-free RPMI-1640 medium and the diluted B7H6 antibody or murine IgG was added to a 96-well round bottom plate in a volume of 25 µL/well.
(3) The rabbit complement (Cedarlane) was dissolved in 1 mL of ultrapure water, then added with 1 mL of a serum-free RPMI-1640 culture medium, and mixed well as a working solution. The complement working solution was added to a 96-well round bottom plate in a volume of 40 µL/well. The cells were incubated at 37°C for 30 min.
(4) Complete RPMI-1640 medium was added to a 96-well round bottom plate at a volume of 110 µL/well and 7-AAD (BD) was added to the 96-well round bottom plate at a volume of 1 µL/well and mixed well. All the liquid in the well was transferred into a flow cytometry tube and detected by using a flow cytometer. The results are shown in FIG. 8. It can be seen that the B7H6 murine 12F5 antibody of the present disclosure has complement dependent cytotoxicity.

### Example 12: B7H6 antibody promotes anti-cancer ability of mice

An in vivo efficacy test was used to detect the function of B7H6 antibody in promoting anticancer in nude mice. Among them, the tumor cells used were acute myeloid leukemia U-937 cells (ATCC No. CRL-1953.2).
(1) Nude mice were implanted subcutaneously in the right flank with 1.5 × 10⁶ U-937 cells per mouse and then randomized into groups.
(2) On days 5, 7, 9, 11, and 13, mice were injected intraperitoneally with 1 mg of antibody per mouse.
(3) Tumor volumes were measured every 2 days.

The results are shown in FIG. 9. It can be seen that the murine antibody 12F5 of the present disclosure has an anticancer function.

It can be seen from the above experimental results that the antibody obtained in the present disclosure is capable of binding to human and monkey B7H6; promoting the interaction between B7H6 and NKp30; and promoting anti-cancer in mice.

The preferred embodiments of the present disclosure have been described in detail above, but the present disclosure is not limited thereto. Within the scope of the inventive concept, a number of simple variants of the inventive solution are possible, including any other suitable combination of the individual features. These simple variants and combinations should likewise be considered as being disclosed as falling within the scope of the present disclosure.

In the description of this specification, the descriptions referring the terms "one embodiment", "some embodiments", "example", "specific examples", or "some examples", etc. mean that a specific feature, structure, material, or characteristic described in connection with the embodiment or example is included in at least one embodiment or example of the present disclosure. In this specification, schematic representations of the terms above do not necessarily refer to the same embodiment or example. Furthermore, the specific features, structures, materials, or characteristics described may be combined in any suitable manner in any one or more embodiments or examples. Further, the different embodiments or examples and the features of the different embodiments or examples described in this specification can be integrated and combined by those skilled in the art without contradicting each other.

While embodiments of the present disclosure have been shown and described, it will be understood that the above-described embodiments are illustrative and should not be construed as limiting the present disclosure, and changes, modifications, substitutions, and alterations may be made to the above-mentioned embodiments by those skilled in the art without departing from the scope of the present disclosure.

## Claims

1. An antibody capable of binding to B7H6 or antigen binding fragment thereof, wherein the antibody or antigen binding fragment thereof comprises a heavy chain CDR1, a heavy chain CDR2, a heavy chain CDR3, a light chain CDR1, a light chain CDR2, and a light chain CDR3, wherein
the heavy chain CDR1 comprises at least one amino acid sequence selected from the group consisting of an amino acid sequence as set forth in SEQ ID NO. 1, and SEQ ID NO. 7, and an amino acid sequence in conservative modification form thereof;
the heavy chain CDR2 comprises at least one amino acid sequence selected from the group consisting of an amino acid sequence as set forth in SEQ ID NO. 2, and SEQ ID NO. 8, and an amino acid sequence in conservative modification form thereof;
the heavy chain CDR3 comprises at least one amino acid sequence selected from the group consisting of an amino acid sequence as set forth in SEQ ID NO. 3, and SEQ ID NO. 9, and an amino acid sequence in conservative modification form thereof;
the light chain CDR1 comprises at least one amino acid sequence selected from the group consisting of an amino acid sequence as set forth in SEQ ID NO. 4, and SEQ ID NO. 10, and an amino acid sequence in conservative modification form thereof;
the light chain CDR2 comprises at least one amino acid sequence selected from the group consisting of an amino acid sequence as set forth in SEQ ID NO. 5, SEQ ID NO. 11, and an amino acid sequence in conservative modification form thereof; and
the light chain CDR3 comprises at least one amino acid sequence selected from the group consisting of an amino acid sequence as set forth in SEQ ID NO. 6, SEQ ID NO. 12, and an amino acid sequence in conservative modification form thereof.

2. The antibody or antigen binding fragment thereof of claim 1, wherein the antibody or antigen binding fragment thereof comprises:
1) the heavy chain CDR1 having an amino acid sequence as set forth in SEQ ID NO. 1, the heavy chain CDR2 having an amino acid sequence as set forth in SEQ ID NO. 2, the heavy chain CDR3 having an amino acid sequence as set forth in SEQ ID NO. 3, the light chain CDR1 having an amino acid sequence as set forth in SEQ ID NO. 4, the light chain CDR2 having an amino acid sequence as set forth in SEQ ID NO. 5, and the light chain CDR3 having an amino acid sequence as set forth in SEQ ID NO. 6: or
2) the heavy chain CDR1 having an amino acid sequence as set forth in SEQ ID NO. 7, the heavy chain CDR2 having an amino acid sequence as set forth in SEQ ID NO. 8, the heavy chain CDR3 having an amino acid sequence as set forth in SEQ ID NO. 9, the light chain CDR1 having an amino acid sequence as set forth in SEQ ID NO. 10, the light chain CDR2 having an amino acid sequence as set forth in SEQ ID NO. 11, and the light chain CDR3 having an amino acid sequence as set forth in SEQ ID NO. 12.

3. The antibody or antigen binding fragment thereof of claim 1 or 2, wherein the antibody or antigen binding fragment thereof comprises a heavy chain variable region and a light chain variable region, (i) the heavy chain variable region comprises an amino acid sequence that is at least 80% homologous to at least one of an amino acid sequence as set forth in SEQ ID NO. 13, SEQ ID NO. 15, SEQ ID NO. 17 and SEQ ID NO. 19 and an amino acid sequence in conservative modification form thereof; and/or, (ii) the light chain variable region comprises an amino acid sequence that is at least 80% homologous to at least one of an amino acid sequence as set forth in SEQ ID NO. 14, SEQ ID NO. 16, SEQ ID NO. 18 and SEQ ID NO. 20 and an amino acid sequence in conservative modification form thereof.

4. The antibody or antigen binding fragment thereof of any one of claims 1-3, wherein the heavy chain variable region comprises an amino acid sequence that is at least 90%, at least 95%, at least 96%, at least 97%, at least 98%, at least 99%, or 100% homologous to the heavy chain variable region selected from (i); wherein the light chain variable region comprises an amino acid sequence that is at least 90%, at least 95%, at least 96%, at least 97%, at least 98%, at least 99%, or 100% homologous to the light chain variable region selected from (ii).

5. The antibody or antigen binding fragment thereof of any one of claims 1-4, wherein the antibody or antigen binding fragment thereof comprises:
1) an amino acid sequence of the heavy chain variable region as set forth in SEQ ID NO. 13, and an amino acid sequence of the light chain variable region as set forth in SEQ ID NO. 14;
2) an amino acid sequence of the heavy chain variable region as set forth in SEQ ID NO. 15, and an amino acid sequence of the light chain variable region as set forth in SEQ ID NO. 16;
3) an amino acid sequence of the heavy chain variable region as set forth in SEQ ID NO. 17, and an amino acid sequence of the light chain variable region as set forth in SEQ ID NO. 18; or
4) an amino acid sequence of the heavy chain variable region as set forth in SEQ ID NO. 19, and an amino acid sequence of the light chain variable region as set forth in SEQ ID NO. 20.

6. The antibody or antigen binding fragment thereof of any one of claims 1-5, wherein the antibody or antigen binding fragment thereof comprises:
1) an amino acid sequence of the heavy chain as set forth in SEQ ID NO. 26, and an amino acid sequence of the light chain as set forth in SEQ ID NO. 27;
2) an amino acid sequence of the heavy chain as set forth in SEQ ID NO. 28, and an amino acid sequence of the light chain as set forth in SEQ ID NO. 29;
3) an amino acid sequence of the heavy chain as set forth in SEQ ID NO. 30, and an amino acid sequence of the light chain as set forth in SEQ ID NO. 31; or
4) an amino acid sequence of the heavy chain as set forth in SEQ ID NO. 32, and an amino acid sequence of the light chain as set forth in SEQ ID NO. 33.

7. The antibody or antigen binding fragment thereof of any one of claims 1-5, wherein the isolated antibody is IgG1, IgG2, or IgG4.

8. The antibody or antigen binding fragment thereof of any one of claims 1-5, wherein the antibody is a monoclonal antibody, murine antibody, chimeric antibody, humanized antibody, human engineered antibody, human antibody, Fv, single chain antibody (scFv), Fab, Fab', Fab'-SH, or F(ab')2.

9. The antibody or antigen binding fragment thereof of any one of claims 1-8, wherein the antibody or antigen binding fragment thereof is capable of binding to the amino acid sequence as set forth in SEQ ID NO. 21.

10. An immunoconjugate, comprising a therapeutic agent and the antibody or antigen binding fragment thereof of any one of claims 1-9 conjugated to the therapeutic agent.

11. A composition, comprising the antibody or antigen binding fragment thereof of any one of claims 1-9, and/or the immunoconjugate of claim 10, and a pharmaceutically acceptable carrier.

12. A kit for detecting B7H6 in a sample, comprising the antibody or antigen binding fragment thereof of any one of claims 1-9.

13. Use of the antibody or antigen binding fragment thereof of any one of claims 1-9 in preparation of a reagent for detection of B7H6 in a sample.

14. Use of the antibody or antigen binding fragment thereof of any one of claims 1-9 in preparation of a medicament for prevention and/or treatment of a B7H6-mediated disease, wherein the B7H6-mediated disease is a cancer or an infectious disease;
optionally, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

15. A nucleic acid, comprising a nucleic acid encoding the antibody or antigen binding fragment thereof of any one of claims 1-9.

16. A recombinant vector or transformant, comprising the nucleic acid of claim 15.

17. A recombinant cell, carrying the nucleic acid of claim 15, the expression vector or transformant of claim 16, or the antibody or antigen binding fragment thereof of any one of claims 1-9.

18. A medicament, comprising the antibody or antigen binding fragment thereof of any one of claims 1-9, the immunoconjugate of claim 10, the composition of claim 11, the nucleic acid of claim 15, the recombinant vector or transformant of claim 16, or the recombinant cell of claim 17.

19. Use of the antibody or antigen binding fragment thereof of any one of claims 1-9, the immunoconjugate of claim 10, the composition of claim 11, the medicament of claim 18, the nucleic acid of claim 15, the recombinant vector or transformant of claim 16 or the recombinant cell of claim 17 in treatment and/or prevention of a B7H6-mediated disease.

20. The use of claim 19, wherein the B7H6-mediated disease is a cancer or an infectious disease;
optionally, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

21. A method for treating and/or preventing a B7H6-mediated disease, comprising administering to a subject at least one of:
1) the antibody or antigen binding fragment thereof of any one of claims 1-9;
2) the immunoconjugate of claim 10;
3) the composition of claim 11;
4) the medicament of claim 18;
5) the nucleic acid of claim 15;
6) the recombinant vector or transformant of claim 16; and
7) the recombinant cell of claim 17.

22. The method of claim 21, wherein the B7H6-mediated disease is a cancer or an infectious disease;
optionally, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

23. A method for diagnosing whether a subject has a B7H6-mediated disease, comprising:
detecting B7H6 in a sample to be tested using at least one of:
1) the antibody or antigen binding fragment thereof of any one of claims 1-9;
2) the nucleic acid of claim 15;
3) the recombinant vector or transformant of claim 16; and
4) the recombinant cell of claim 17; and
determining the content of B7H6 in the sample to be tested based on a result of detecting of the B7H6.

24. The method of claim 23, wherein the content of B7H6 in the sample to be tested that is not lower than the minimum standard for disease is an indication that the sample to be tested is derived from a patient having a B7H6-mediated disease.

25. The method of claim 23, wherein the B7H6-mediated disease is a cancer or an infectious disease;
optionally, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.

26. Use of at least one of the following in diagnosing whether a subject has a B7H6-mediated disease:
1) the antibody or antigen binding fragment thereof of any one of claims 1-9;
2) the nucleic acid of claim 15;
3) the recombinant vector or transformant of claim 16; and
4) the recombinant cell of claim 17.

27. The use of claim 26, wherein the B7H6-mediated disease is a cancer or an infectious disease;
optionally, the cancer is at least one of the lung cancer, liver cancer, ovarian cancer, cervical cancer, skin cancer, bladder cancer, colon cancer, breast cancer, glioma, kidney cancer, stomach cancer, esophageal cancer, oral squamous cell cancer, and head and neck cancer.
